(19)

Europäisches Patentamt
European Patent Office
Office européen des brevets

(11) **EP 4 045 658 B1**

(12) **EUROPEAN PATENT SPECIFICATION**

(45) Date of publication and mention
of the grant of the patent:
**24.09.2025  Bulletin 2025/39**

(21) Application number: **20876950.5**

(22) Date of filing: **15.10.2020**

(51) International Patent Classification (IPC):
*C12N 15/55* (2006.01)      *C40B 40/02* (2006.01)
*C12Q 1/6869* (2018.01)     *C12N 15/10* (2006.01)
*C12Q 1/6806* (2018.01)

(52) Cooperative Patent Classification (CPC):
(C-Sets available)
**C40B 40/06; C12N 9/22; C12N 15/1058;
C12N 15/1075; C12Q 1/6806; C40B 40/08;**
C12N 2310/20                                (Cont.)

(86) International application number:
**PCT/SG2020/050588**

(87) International publication number:
**WO 2021/076053 (22.04.2021 Gazette 2021/16)**

(54) **ASSAYS FOR MEASURING NUCLEIC ACID MODIFYING ENZYME ACTIVITY**

ASSAYS ZUM MESSEN DER AKTIVITÄT VON NUKLEINSÄUREMODIFIZIERENDEN ENZYMEN

DOSAGES POUR MESURER L'ACTIVITÉ ENZYMATIQUE MODIFIANT L'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.10.2019  SG 10201909632P**

(43) Date of publication of application:
**24.08.2022  Bulletin 2022/34**

(73) Proprietor: **Agency for Science, Technology and
Research**
**Singapore 138632 (SG)**

(72) Inventors:
• **ONG, Ting Xun Nicholas**
**Singapore 138672 (SG)**
• **CHEW, Wei Leong**
**Singapore 138672 (SG)**

(74) Representative: **Maiwald GmbH**
**Elisenhof**
**Elisenstraße 3**
**80335 München (DE)**

(56) References cited:
**WO-A1-2018/118968      WO-A1-2018/118968
WO-A1-2018/149888      WO-A1-2019/168953**

• **DONNA J. PALMER ET AL: "Production of
CRISPR/Cas9-Mediated Self-Cleaving Helper-
Dependent Adenoviruses", MOLECULAR
THERAPY- METHODS & CLINICAL
DEVELOPMENT, vol. 13, 1 June 2019
(2019-06-01), GB, pages 432 - 439, XP055761603,
ISSN: 2329-0501, DOI: 10.1016/
j.omtm.2019.04.003**

Remarks:
The complete document including Reference
Table(s) and the Sequence Listing(s) can be
downloaded from the EPO website

EP 4 045 658 B1

**(Cont. next page)**

(52) Cooperative Patent Classification (CPC): (Cont.)

C-Sets
**C12N 15/1075, C12Q 2521/301, C12Q 2535/122,
C12Q 2563/159, C12Q 2565/631;
C12Q 1/6806, C12Q 2521/301, C12Q 2535/122,
C12Q 2563/159, C12Q 2565/631**

**Description**

**FIELD OF THE INVENTION**

The present invention relates to the field of biotechnology, specifically the development of multiplex assays suitable for measuring enzymatic activities.

**BACKGROUND OF THE INVENTION**

Nucleic acid modifying enzymes such as zinc finger nucleases (ZFNs), transcription activator-like effector nucleases (TALENs), and clustered regularly-interspersed short palindromic repeat (CRISPR)-associated nucleases have become invaluable, both as tools in biomedical research and in biotechnology industry. As therapeutic modalities, these nucleic acid modifying enzymes could treat previously incurable genetic diseases by directly modifying DNA or RNA. In order to realize the vast industrial and medical potentials of nucleic acid modifying enzymes, limitations in the naturally occurring components have to be addressed. These limitations include issues with targeting efficiency, targeting specificity, immunogenicity, and compatibility with delivery vectors and function-conferring protein fusion moieties. To address these limitations, the enzymes have to be modified and assayed for enzymatic activity, in a process called protein engineering. Enzymes such as CRISPR-Cas can also be engineered to have enhanced functionality (e.g. more specific for its targets, more efficient in targeting) and/or to have novel functions (e.g. base-editing, immune-evading, epigenetic-modifying), either by changing the protein amino acid sequence or by fusing/colocalising function-conferring protein domains onto the Cas protein or CRISPR complex.

A general approach for engineering an enzyme begins with (i) designing and creating a library of DNA variants encoding many different sequences of the enzyme (with amino acid change(s) compared to the naturally occurring wildtype), (ii) expressing these variants in compartments, such as in cells or *in vitro,* (iii) measuring enzyme activity or linking enzyme activity via downstream biochemical reactions or cellular phenotypes, followed by, either "screening" (whereby no selection pressure is applied to segregate the active and inactive variants) or "selecting" (whereby selection pressure is applied to segregate the active and inactive variants). Protein engineering, specifically of programmable endonucleases like CRISPR-Cas, have been performed largely by the latter "selection" approach. This approach biases active variants in a binary fashion (cells survive when expressing active versions of the protein, die when expressing inactive versions of the protein; also called positive selection), and do not provide information of the degree of protein activity (e.g. doesn't discriminate between highly active proteins versus one that is half as active), nor consider/provide information on the inactive protein variants. Negative selection can also be conducted, whereby only the inactive variants are retained and identified, and the active variants are depleted and not directly measured. In both cases, the test of activity is linked to and manifested by enrichment/depletion of library members. The "screening" approach is not scalable, because of the increased resources needed to maintain and measure both active and inactive variants. Hence, the engineering and assaying of nucleic acid modifying enzymes such as CRISPR-Cas proteins have been limited both in terms of numbers of variants testable and numbers of mutation possible per variant. CRISPR-Cas proteins can be engineered to perform better, faster, and safer with multiple amino acid substitutions engineered into the protein, but current approaches do not allow exploration of this functional space.

International patent publication WO 2018/118968 A1 describes methods and systems for characterization of nuclease activity using compartmentalized *in vitro* transcription and translation.

Palmer et al. (Palmer D. J., Turner D. L., Ng P. Production of CRISPR/Cas9-Mediated Self-Cleaving Helper-Dependent Adenoviruses. Mol Ther Methods Clin Dev. 2019; 13: 432-439) describe a system to produce helper-dependent adenoviruses that express CRISPR/Cas9 to direct cleavage of the vectors' own genome after transduction of target cells.

There is thus a need for a high-throughput screening technology to detect and identify functional variants from millions to billions and beyond of enzyme library candidates that can still recognize, cleave, or modify their nucleic acid targets precisely and efficiently. Such a technology would enable the screening and engineering of novel nucleic acid modifying enzymes, as well as the screening and optimization of other factors affecting the enzymatic activities, such as guide RNAs and target sequences. The object of the present invention is therefore to provide an improved method which addresses the above needs.

**SUMMARY OF THE INVENTION**

The invention is defined by the appended claims.

In one aspect, the present disclosure refers to a method comprising the steps of:

a) segregating a plurality of polynucleotide constructs into compartments, wherein each compartment comprises a single polynucleotide construct, wherein each polynucleotide construct comprises

> i) a first polynucleotide sequence encoding a nucleic acid modifying enzyme or a variant thereof, operably linked to a first promoter; and
> ii) a second polynucleotide sequence comprising a DNA target or a DNA template encoding an RNA target, wherein when the second polynucleotide sequence comprises a DNA template encoding an RNA target, said RNA target is co-expressed contiguously with the nucleic acid modifying enzyme as a single RNA transcript, driven by the first promoter;

and wherein the plurality of the polynucleotide constructs encode different variants of the nucleic acid modifying enzyme, and/or different DNA or RNA targets;
b) subjecting the compartments to conditions which allow *in vitro* expression of RNAs and proteins;
c) subjecting the plurality of the compartments to conditions which allow the modification of DNA/RNA targets by nucleic acid modifying enzymes which have modification activity towards said DNA or RNA targets, thereby producing a population of DNA/RNA molecules that comprises one or more of the following:

> i. polynucleotide constructs and/or RNA transcripts or fragments thereof that have been modified by the nucleic acid modifying enzyme(s);
> ii. polynucleotide constructs and/or RNA transcripts which have not been modified by the nucleic acid modifying enzyme(s);

d) harvesting the population of DNA/RNA molecules produced in step (c) and subjecting the same to single molecule sequencing;
e) detecting and counting the DNA/RNA molecules referred to in step c)i and c)ii based on the sequencing results.

In another aspect, the present disclosure refers to a method comprising the steps of:

a) segregating a plurality of polynucleotide constructs into compartments, wherein each compartment comprises a single polynucleotide construct, wherein each polynucleotide construct comprises:

> i) a first polynucleotide sequence encoding a guide RNA (gRNA) operably linked to a first promoter;
> ii) a second polynucleotide sequence comprising a DNA target or a DNA template encoding an RNA target, wherein when the second polynucleotide sequence comprises a DNA template encoding an RNA target, said RNA target is co-expressed contiguously with the gRNA as a single RNA transcript, driven by the first promoter;

wherein the plurality of the polynucleotide constructs encode different gRNAs, and/or different DNA or RNA targets; and wherein each compartment further comprises an RNA-guided nucleic acid modifying enzyme or a variant thereof or a nucleotide template encoding the same;
b) subjecting the compartments to conditions which allow in vitro transcription and/or translation of RNAs and proteins;
c) subjecting the compartments to conditions which allow the modification of DNA and/or RNA targets by RNA-guided nucleic acid modifying enzymes which have functional activity towards said DNA or RNA targets in the presence of a gRNA, thereby producing a population of DNA/RNA molecules that comprises one or more of the following:

> i. polynucleotide constructs and/or RNA transcripts or fragments thereof that have been modified by the nucleic acid modifying enzyme(s);
> ii. polynucleotide constructs and/or RNA transcripts which have not been modified by the nucleic acid modifying enzyme(s);

d) harvesting the population of DNA/RNA molecules produced in step (c) and subjecting the same to single molecule long-read sequencing;
e) detecting and counting the DNA/RNA molecules referred to in step c)i and/or c)ii based on the sequencing results.

## BRIEF DESCRIPTION OF THE DRAWINGS

The invention will be better understood with reference to the detailed description when considered in conjunction with the non-limiting examples and the accompanying drawings, in which:

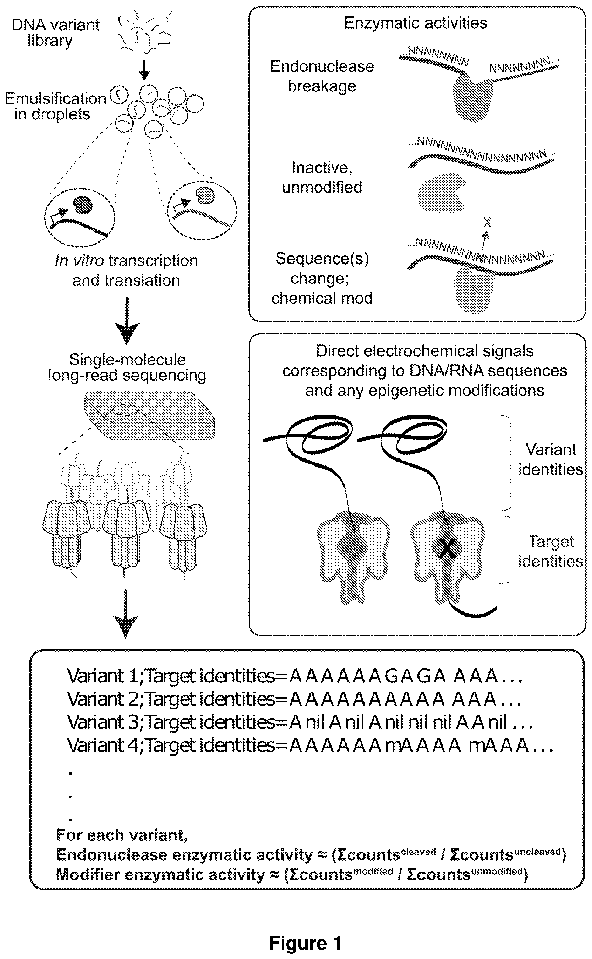

**Figure 1.** Diagram illustrating a non-limiting list of the key concepts and steps of this invention, wherein the compartmentalization occurs via generation of water-in-oil emulsion droplets.

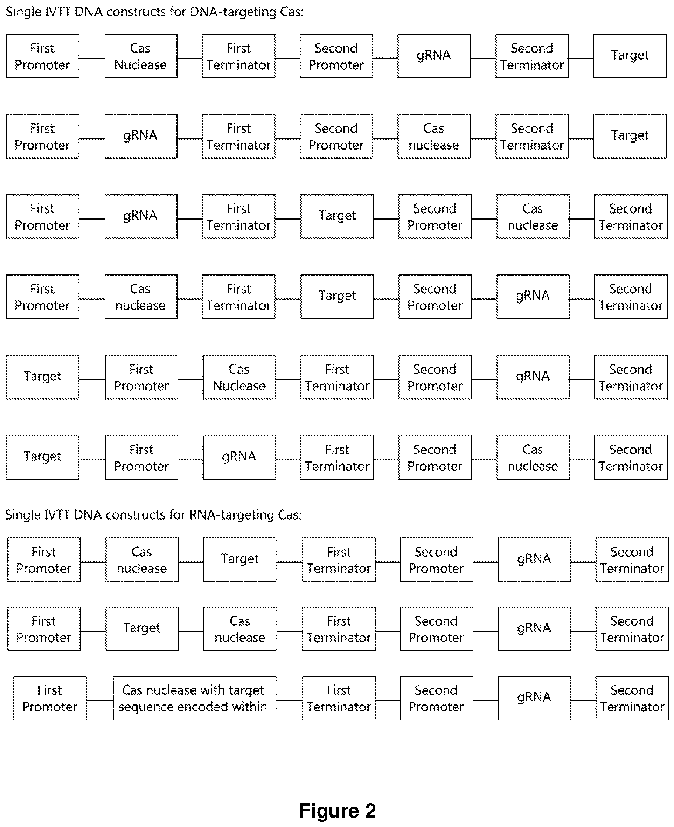

**Figure 2.** Diagram illustrating non-limiting examples of polynucleotide constructs as disclosed in the present disclosure. Note that 'Cas nuclease' can be replaced with any nucleic acid modifying enzyme, and can also refer to Cas variants such as inactivated Cas nuclease, or Cas protein fused to or associated with function-conferring domains.

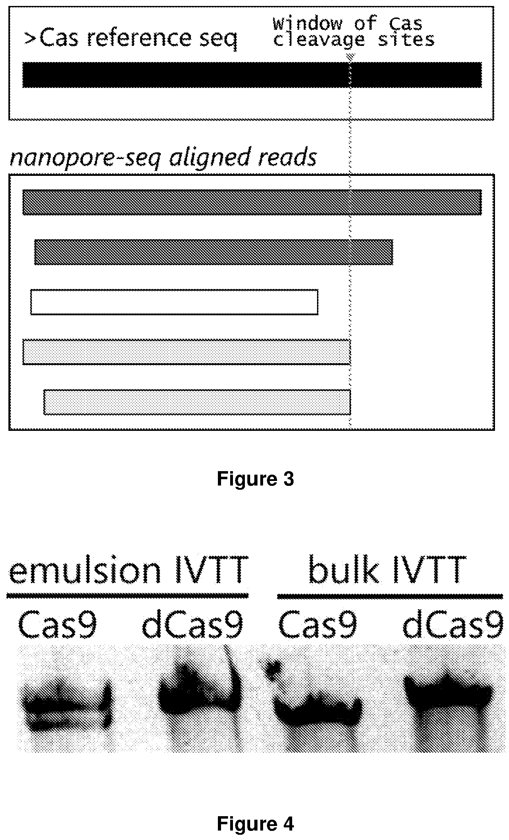

**Figure 3.** A diagrammatic representation of how DNA/RNA molecule reads are counted for the calculation of enzymatic activity, in one example where the enzyme is a Cas nuclease and the modification is DNA cleavage. In this example, the DNA target site resides 3' of the encoded Cas variant. The nanopore-sequencing reads (aligned against a reference sequence) with aligned 3' ends that map to the reference sequence at sites 3' downstream of the window of expected Cas cleavage sites are considered uncleaved (dark grey bars of "nanopore-seq aligned reads"; Figure 3), while read alignments with 3' ends lying within the window of Cas cleavage sites are considered cleaved (light grey bars of "nanopore-seq aligned reads"; Figure 3), and reads that don't fulfill either criteria are discarded as non-informative as these cannot be empirically determined if they were cleaved or not (white bar of "nanopore-seq aligned reads"; Figure 3).

**Figure 4.** Gel visualization of purified IVTT Sp Cas9 and dCas9 DNA constructs from compartmentalized (via emulsion) IVTT reactions vs. bulk IVTT reactions. 750 ng of Sp Cas9 construct with IVTT reagents (New England Biolabs PURExpress #E6800) were mixed on ice to produce a 75 $\mu$L IVTT aqueous mixture. 50 $\mu$L of the aqueous mixture was added in 5 aliquots of 10 $\mu$L over 2 minutes to the oil surfactant mix on ice while the stir bar was spinning at 1150 rpm to generate an emulsion mixture. The emulsion mixture was allowed to continue mixing for an additional minute on ice. In one example, the emulsion mixture was then subjected to homogenization (8000 rpm for 3 minutes; IKA Ultraturrax T10 homogenizer) to create a more monodisperse distribution of emulsion droplet sizes. The remaining 25 $\mu$L of the aqueous mixture was kept on ice for a bulk IVTT reaction as a control. This was repeated for a Sp dCas9 construct as well. The emulsion and bulk IVTT mixtures were then incubated for 4 h at 37 °C for IVTT to proceed, followed by 65 °C for 15 min to inactivate the proteins. The DNA from all the IVTT reactions were then purified individually and aliquots were visualized on an agarose gel after size separation via gel electrophoresis. This data shows that the IVTT reagents successfully transcribe and translate proteins both in bulk reactions and in emulsion droplets.

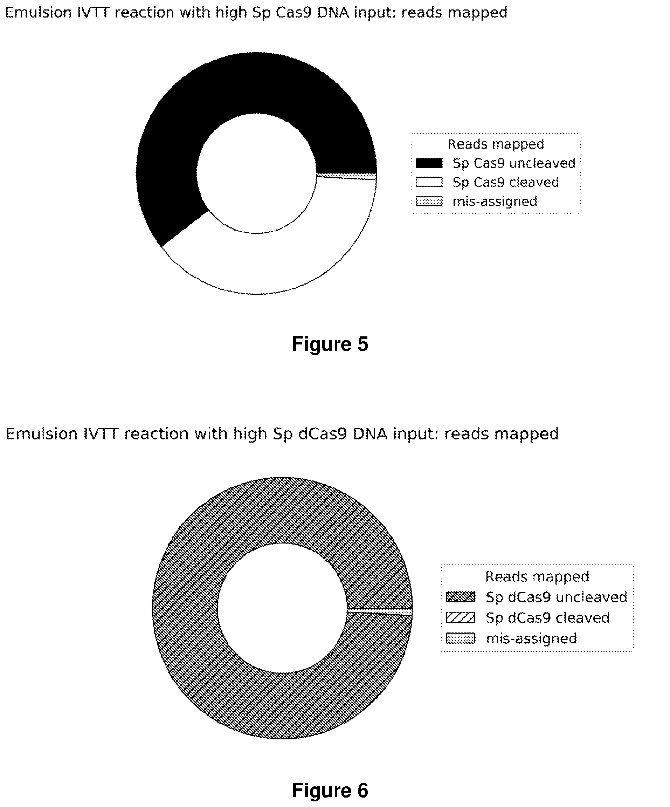

**Figure 5.** Nanopore-sequencing reads from an emulsion IVTT self-cleaving assay, with high input concentration of Sp Cas9 construct. A small subset of reads in this sublibrary were mapped to Sp dCas9 and thus classified as mis-assigned (light grey section; Figure 5) on the plot since only Sp Cas9 DNA was provided as the input for this emulsion IVTT reaction. The Sp Cas9 emulsion IVTT nanopore sequencing reads show a mix of cleaved and uncleaved construct fragments detected (white and black sections respectively; Figure 5). This data thus show that the nanopore single molecule sequencing can detect both modified and unmodified polynucleotide constructs (products of enzymatic activity or inactivity) from emulsion IVTT reactions.

**Figure 6.** Nanopore-sequencing reads from an emulsion IVTT self-cleaving assay, with high input concentration of Sp dCas9 construct. Reads that failed to pass an alignment quality filter were classified accordingly. The Sp dCas9 emulsion IVTT nanopore sequencing reads show up overwhelmingly as uncleaved construct fragments as expected (grey section with stripes; Figure 6). A small subset of reads in this sub-library were mapped to Sp Cas9 and thus classified as mis-assigned (light grey section; Figure 6) on the plot since only Sp dCas9 DNA was provided as the input for this emulsion IVTT reaction. This result supports the robustness of the method, as the inactivity of the Sp dCas9 is accurately detected and measured by the sequencing reads.

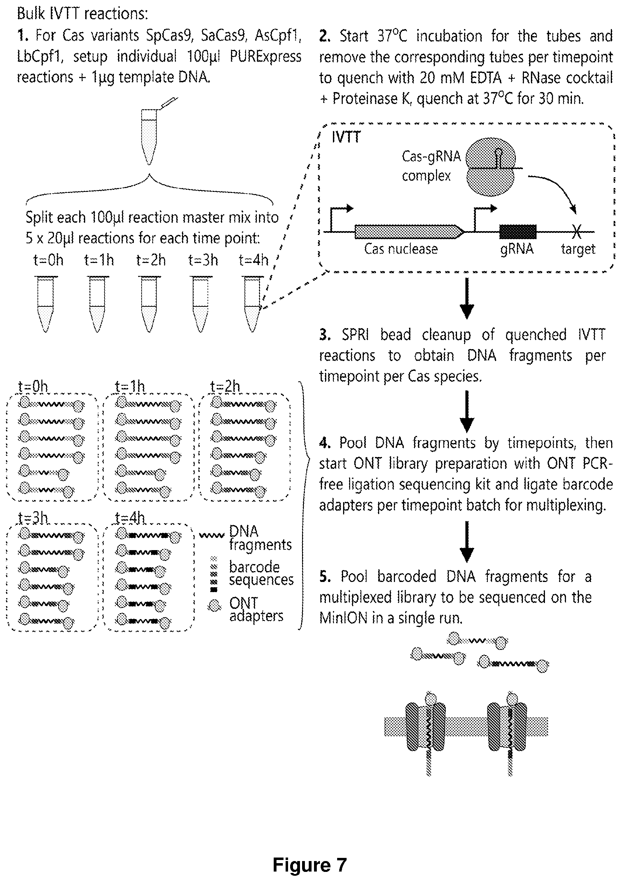

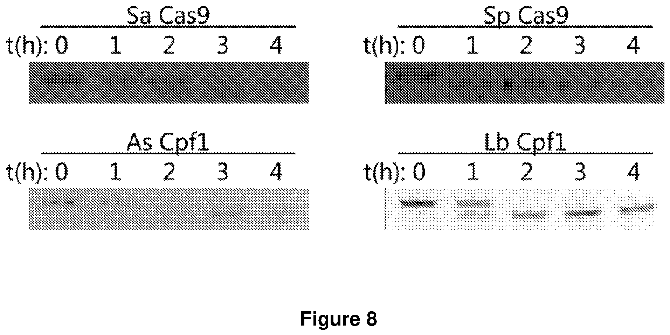

**Figure 7.** Diagram depicting an exemplary workflow for bulk IVTT and self-cleaving assay time course experiment with a nanopore-sequencing readout of results. In this example, bulk IVTT reactions were set up on ice for different CRISPR-Cas constructs (e.g. Sp Cas9, Sa Cas9, As Cpf1, Lb Cpf1) which all shared a similar arrangement of components as described in the nucleic acid template sequence above. These were then divided equally into 5 corresponding aliquots for each time point (Figure 7 Part 1). These bulk IVTT aliquots were then incubated at 37 °C and removed per designated time point to be quenched with EDTA inhibitor and enzymes to stop the IVTT reactions and Cas cleavage of encoding DNA constructs (Figure 7 Part 2). The quenched IVTT reactions were then processed with SPRIselect beads cleanup to purify the DNA fragments (Figure 7 Part 3). Small aliquots of these DNA fragments of the different Cas orthologs from different IVTT timepoints were then visualized on an agarose gel after size separation via gel electrophoresis, as seen in Figure 8 below. The remaining aliquots of purified DNA fragments were then pooled by their respective time points but irrespective of Cas species i.e. DNA fragments for Sp Cas9, Sa Cas9 etc. at each timepoint were mixed together and were barcoded individually using the ONT EXP-NBD104 PCR-Free native barcoding expansion kit (Figure 7 Part 4) to multiplex these pooled sublibraries for a single nanopore sequencing run (Figure 7 Part 5). The nanopore sequencing results were then filtered for quality and analyzed using publicly available bioinformatics tools, followed by the analytic approach disclosed herein.

**Figure 8.** Gel visualization of purified IVTT constructs of different CRISPR-Cas orthologs from bulk IVTT reactions after step shown in Figure 7 Part 3. This data shows that different Cas proteins (variants or orthologs) are successfully transcribed and translated in the bulk reactions.

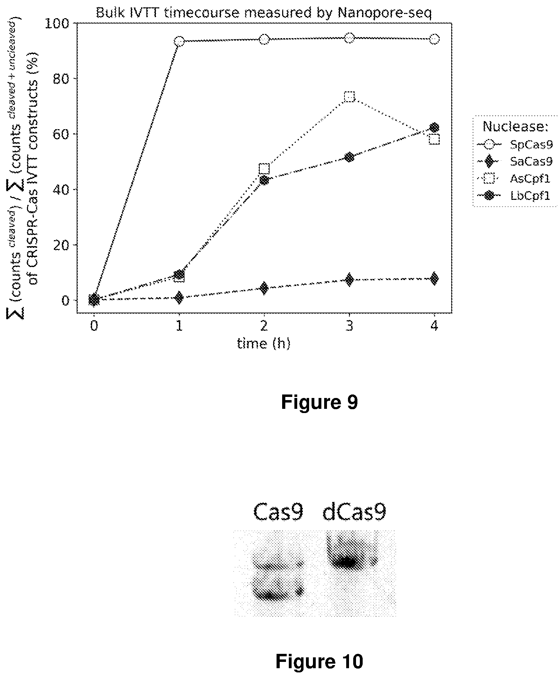

**Figure 9.** Plot of Cas-encoding DNA fragments detected by nanopore-sequencing from a bulk IVTT and self-cleaving assay timecourse experiment. This data shows that single molecule sequencing can detect enzymatic products and measure enzymatic activities of different nucleic acid modifying enzymes in a multiplex manner.

**Figure 10.** Gel visualization of purified IVTT Sp Cas9 and dCas9 DNA constructs from bulk IVTT reactions. 500 ng of Sp Cas9 (sequence as depicted in above) with IVTT reagents (New England Biolabs PURExpress #E6800) on ice to produce a 50 μL IVTT aqueous mixture. The same was done for a Sp dCas9 construct as well; the Sp dCas9 construct contains an essentially identical DNA sequence to that of the Sp Cas9 construct, except for 2 deactivating mutations in the Sp Cas9 gene (D10A and H840A) to yield a Sp dCas9 gene. These 50 μL bulk IVTT reactions were incubated at 37 °C for 4h for IVTT to proceed, followed by 65 °C for 15 min to inactivate the proteins. 20 mM EDTA (pH 8.0) inhibitor with RNase cocktail and Proteinase K were added to the bulk IVTT reactions to remove excess RNA and proteins from the IVTT reaction at 37 °C for 30 min. The DNA (polynucleotide constructs) from both bulk IVTT reactions were then purified individually with SPRIselect paramagnetic beads, aliquots of which were then visualized on an agarose gel after size separation via gel electrophoresis. This data shows that the Cas proteins are successfully transcribed and translated in bulk IVTT reactions.

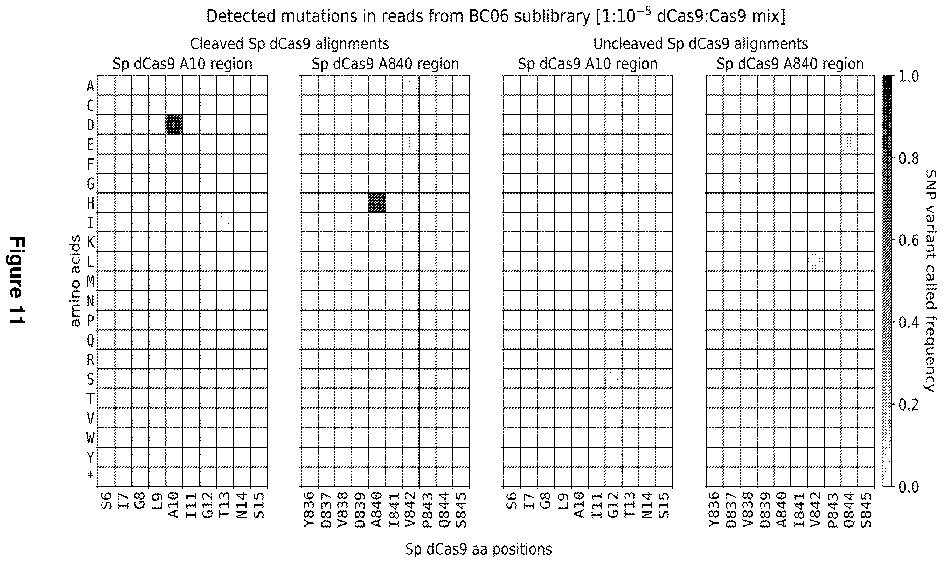

**Figure 11.** Demonstration of the direct detection and counting of polynucleotides which have been modified or have not been modified by the nucleic acid modifying enzymes. The Sp Cas9 and Sp dCas9 DNA constructs purified from the bulk IVTT reactions of which gel visualizations were depicted in Figure 10 were mixed together in different ratios. These mixtures of purified DNA constructs were then prepared for nanopore-sequencing. By aligning all the nanopore-sequencing reads against a Sp dCas9 construct reference sequence, the presence of cleaved Sp Cas9 reads is detected using bioinformatics tools that a person having ordinary skill in the art of sequencing data analysis could conduct. This workflow enables the detection of variations (indels - insertions and deletions or SNPs - single nucleotide polymorphisms) in the sequenced reads aligned against a reference sequence; specific interest was placed in the detection of SNPs that represent the expected sequence differences between the otherwise identical Sp dCas9 and Sp Cas9 constructs, namely the D10A and H840A catalytically deactivating mutations in Sp dCas9 versus Sp Cas9. Raw nanopore-sequencing read alignments were categorized as cleaved versus uncleaved by sequence mapping against a Sp dCas9 reference sequence as depicted in Figure 3, then processed for SNP detections that resulted in an amino acid residue change. The Sp Cas9 sequence on each filtered read alignment was translated into its corresponding amino acid sequence, and detected SNPs that resulted in an amino acid change from the Sp dCas9 reference amino acid sequence was counted. In the plot above, detected SNPs are represented in the heatmaps for selected regions of interest in the Sp dCas9 reference that contain the D10A and H840A catalytically deactivating mutations in Sp dCas9. Reads categorized as cleaved (2 subplots on the left; Figure 11) were enriched for SNPs that corresponded to possessing D10 and H840 residues (dark grey squares in the heatmap; Figure 11) i.e. these cleaved reads contained the catalytically active Sp Cas9 sequence. Other SNPs detected that resulted in amino acid mutations represented in the plot above with much lighter grey squares in the heatmap are false positives that arose from raw sequencing errors inherent in currently available nanopore sequencing technology. This data demonstrates the detection of cleaved and uncleaved Sp Cas9 DNA fragments which could be distinguished from the detection of the uncleaved Sp dCas9 DNA fragments in the raw nanopore sequencing data. Notably, the method is able to detect the presence of cleaved Sp Cas9 DNA fragments even in the 1:$10^{-5}$ mix of purified Sp dCas9 and Sp Cas9 bulk IVTT DNA products respectively (Figure 11).

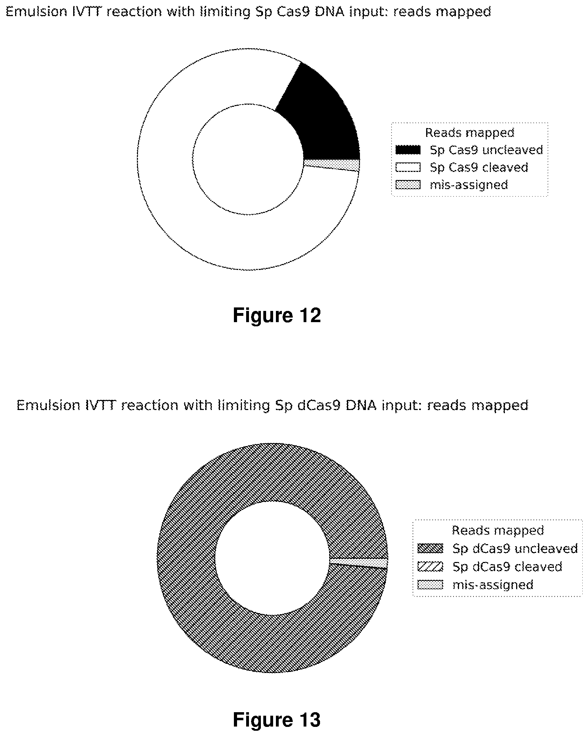

**Figure 12.** Nanopore-sequencing reads from an emulsion IVTT self-cleaving assay, with limiting input concentration of Sp Cas9 construct. As expected of Sp Cas9 enzyme, the emulsion IVTT nanopore sequencing reads show a mix of cleaved (white section; Figure 12) and uncleaved (black section; Figure 12) construct fragments detected. This data thus supports the robustness of the assay wherein IVTT and enzymatic reactions are performed in the emulsion droplets. A small subset of reads in this sublibrary were mapped to Sp dCas9 and thus classified as mis-assigned (light grey section; Figure 12) on the plot since only Sp Cas9 DNA was provided as the input for this emulsion IVTT reaction.

**Figure 13.** Nanopore-sequencing reads from an emulsion IVTT self-cleaving assay, with limiting input concentration of Sp dCas9 construct. The Sp dCas9 emulsion IVTT nanopore sequencing reads show up overwhelmingly as uncleaved (grey section with stripes; Figure 13) construct fragments, demonstrating that the Sp dCas9 is inactive majority of the time, as expected. This data thus also supports the robustness of the assay wherein IVTT and enzymatic reactions are performed in the emulsion droplets. A small subset of reads in this sublibrary were mapped to Sp Cas9 and thus classified as mis-assigned (light grey section; Figure 13) on the plot since only Sp dCas9 DNA was provided as the input for this emulsion IVTT reaction.

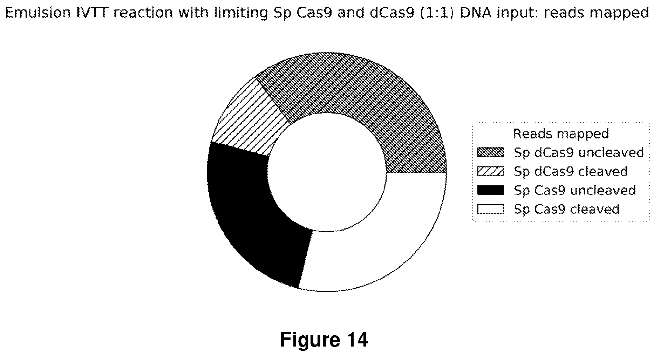

**Figure 14.** Nanopore-sequencing reads from an emulsion IVTT self-cleaving assay, with limiting input concentration of Sp Cas9 and Sp dCas9 constructs provided at an equimolar ratio. The nanopore sequencing reads show an approximately equal distribution of Sp Cas9 and Sp dCas9 mapped reads as expected. Further, the Sp Cas9 mapped reads show a nearly equal split of cleaved and uncleaved fragments (white and black sections respectively; Figure 14), while a large majority of Sp dCas9 mapped reads are classified as uncleaved (grey section with stripes; Figure 14). This data thus further demonstrates that method disclosed herein can measure the enzymatic activities of different variants (in this example Cas variants, but the method may also be used to screen variants of other components of the enzymatic reaction such as the target or the gRNA).

**DEFINITIONS**

Several terms that are employed throughout the specification are defined in the following paragraphs. Other definitions may also found within the body of the specification.

As used herein, the terms "about" and "approximately," in reference to a number, is used herein to include numbers that fall within a range of 20%, 10%, 5%, 2.5%, 2%, 1.5% or 1% in either direction (greater than or less than) of the number unless otherwise stated or otherwise evident from the context (except where such number would exceed 100% of a possible value).

The terms "polynucleotide", "nucleic acid" and "oligonucleotide" are used interchangeably and refer to a polymeric form of nucleotides of any length, either deoxyribonucleotides or ribonucleotides or analogues thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogues. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labelling component. The term also refers to both double- and single-stranded molecules. Unless otherwise specified or required, a polynucleotide encompasses both the double-stranded form and each of two complementary single-stranded forms known or predicted to make up the double-stranded form. As used herein, the term "polypeptide" generally has its art-recognized meaning of a polymer of amino acids. The term is also used to refer to specific functional classes of polypeptides, such as, for example, nucleases, antibodies, etc.

The term "operably linked", as used herein, refers to a juxtaposition wherein the components described are in a relationship permitting them to function in their intended manner. A control element, e.g., a promoter, "operably linked" to a functional element is associated in such a way that expression and/or activity of the functional element is achieved under conditions compatible with the control element. In some embodiments, "operably linked" control elements are contiguous (e.g., covalently linked) with the coding elements of interest; in some embodiments, control elements act in *trans* to or otherwise at the functional element of interest.

The term "nucleic acid modifying enzyme" refers to a macromolecular biological catalyst which may be a protein or nucleic acid in nature, and is capable of modifying a nucleic acid. The term "RNA-guided nucleic acid modifying enzyme" broadly refer to an enzyme that interacts or forms a complex with a guide RNA, and can specifically target or bind with a polynucleotide of a specific sequence which usually comprises a sequence complementary to the targeting domain of the gRNA. Upon binding with the target polynucleotide, the RNA-guided nucleic acid modifying enzyme may remain bound with the target polynucleotide, or it may cleave the target polynucleotide if the RNA-guided nucleic acid modifying enzyme is a nuclease; or it may modify the polynucleotide in other manners if it has a functional domain to do so. In one example, the RNA-guided nucleic acid modifying enzyme is a CRISPR-associated protein (Cas). Many Cas proteins possess endonuclease activity and are also termed Cas nucleases. In a specific example, the RNA-guided nucleic acid modifying enzyme is selected from the group consisting of a Cas3, a Cas9, a Cas10, a Cas12a (also known as Cpf1), a Cas13a (also known as C2c2), a Cas13b, a Cas13c, a Cas13d, a Cas14, a CasX, a CasΦ and variants thereof.

The terms "guide RNA" and "gRNA" refer to any nucleic acid that promotes the specific association (or "targeting") of an RNA-guided nucleic acid modifying enzyme to a target sequence either in a cell or in a cell free environment. gRNAs can be unimolecular (comprising a single RNA molecule, and referred to alternatively as chimeric), or modular (comprising more than one, and typically two, separate RNA molecules, such as a crRNA and a tracrRNA, which are usually associated with one another, for instance by duplexing).

As used herein, the term "target" (or "target site") refers to a nucleic acid sequence that defines a portion of a nucleic acid (or polynucleotide) to which a binding molecule will bind, provided sufficient conditions for binding exist. In some embodiments, a target site is a nucleic acid sequence to which a nucleic acid modifying enzyme described herein binds and/or that is modified by such nucleic acid modifying enzyme. In some embodiments, a target is a nucleic acid sequence to which a guide RNA described herein binds. A target may be single-stranded or double-stranded. The nucleic acid modifying enzymes as disclosed herein may modify DNA or RNA. Therefore, the "target" may be a DNA sequence or an RNA sequence, and is referred to as a "DNA target" and a "RNA target" respectively. In the context of nucleases that dimerize, for example, nucleases comprising a Fokl DNA cleavage domain, a target typically comprises a left-half site (bound by one monomer of the nuclease), a right-half site (bound by the second monomer of the nuclease), and a spacer sequence between the half sites in which the cut is made. In some embodiments, the left-half site and/or the right -half site is between 10-18 nucleotides long. In some embodiments, either or both half- sites are shorter or longer. In some embodiments, the left and right half sites comprise different nucleic acid sequences. In the context of zinc finger nucleases, a target may, in some embodiments, comprise two half-sites that are each 6-18 bp long flanking a non-specified spacer region that is 4-8 bp long. In the context of TALENs, target may, in some embodiments, comprise two half-sites sites that are each 10-23 bp long flanking a non-specified spacer region that is 10-30 bp long. In the context of RNA-guided (e.g., RNA-programmable) nucleic acid modifying enzymes, a target typically comprises a nucleotide sequence (e.g. the "protospacer" in CRISPR-Cas) that is complementary to a guide RNA (gRNA), and a protospacer adjacent motif (PAM) at the 3' end or 5' end adjacent to the guide RNA-complementary sequence. For CRISPR-Cas enzymes which target RNA (e.g. the Cas13 family), the RNA target may comprise a Protospacer Flanking Sequence (PFS) instead of the PAM sequence. The DNA or RNA target of Cas enzymes may comprise, in some embodiments, 16-24 nucleotides in length that are complementary to the gRNA, and a 3-6 base pair PAM/PFS (e.g., NNN, wherein N represents any nucleotide).

"Binding" as used herein refers to a non-covalent interaction between macromolecules (e.g., between a protein and a polynucleotide).

"Modifying" of a polynucleotide refers to any chemical or physical changes to the components or structure of the polynucleotide, which includes the breaking/cleaving the polynucleotide, creating a nick (single strand breakage) in a double stranded polynucleotide, substituting one or more nucleotide bases, inserting or deleting one or more nucleotide bases, or covalently modifying nucleotide bases with chemical and epigenetic markers (such as cytosine methylation and hydroxymethylation).

As used herein, the term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a polypeptide may have a characteristic sequence element comprising a plurality of amino acids having designated positions relative to one another in linear or three-dimensional space and/or contributing to a particular biological function; a nucleic acid may have a characteristic sequence element comprised of a plurality of nucleotide residues having designated positions relative to on another in linear or three-dimensional space. For example, a variant polypeptide may differ from a reference polypeptide as a result of one or more differences in amino acid sequence and/or one or more differences in chemical moieties (e.g., carbohydrates, lipids, etc.) covalently attached to the polypeptide backbone. In some embodiments, a variant polypeptide shows an overall sequence identity with a reference polypeptide (e.g., a nucleic acid modifying enzyme described herein) that is at least 60%, 65%, 70%, 75%, 80%, 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide, e.g., enzymatic activity. In some embodiments, a variant polypeptide lacks one or more of the biological activities of the reference polypeptide. In some embodiments, a variant polypeptide shows a reduced level of one or more biological activities (e.g., enzymatic activity) as compared with the reference polypeptide. In some embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (e.g., fewer than 5, 4, 3, 2, or 1) of substituted functional residues (i.e., residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature.

The term "library", as used herein in the context of nucleic acids or proteins, refers to a population of two or more different polynucleotide constructs or proteins, respectively. In some embodiments, a library of polynucleotide constructs comprises at least two polynucleotide constructs comprising different sequences encoding nucleic acid modifying enzymes, at least two polynucleotide constructs comprising different sequences encoding guide RNAs, at least two polynucleotide constructs comprising different PAMs, and/or at least two nucleic acid molecules comprising different target sites. In some examples, a library comprises at least $10^1$, at least $10^2$, at least $10^3$, at least $10^4$, at least $10^5$, at least $10^6$, at least $10^7$, at least $10^8$, at least $10^9$, at least $10^{10}$, at least $10^{11}$, at least $10^{12}$, at least $10^{13}$, at least $10^{14}$, or at least $10^{15}$

different nucleic acid templates. In some embodiments, the members of the library may comprise randomized sequences, for example, fully or partially randomized sequences. In some embodiments, the library comprises nucleic acid molecules that are unrelated to each other, e.g., nucleic acids comprising fully randomized sequences. In other embodiments, at least some members of the library may be related, for example, they may be variants or derivatives of a particular sequence.

As used herein, the term "expression" of a nucleic acid sequence refers to the generation of any gene product from the nucleic acid sequence. In some examples, a gene product can be an RNA transcript. In some embodiments, a gene product can be a polypeptide. In some embodiments, expression of a nucleic acid sequence involves one or more of the following: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

The term "compartments", as used herein in the context of segregating polynucleotide constructs into compartments, may refer to any physical or virtual compartments such as emulsion droplets and nanowells, and virtual compartments such as microfluidic or hydrogel enabled segregation of reagents and reactions.

The term "promoter" as used herein refers to transcription promoters which confer accurate transcription initiation. The promoter as used herein includes any promoters which can be used to produce mRNA encoding a protein (such as Cas protein) or an RNA transcript (such as a guide RNA). In some examples, the promoter is compatible with cell-free in vitro transcription and translation reactions. Examples of promoters which may be used in the context of this invention include but are not limited to: a T7 promoter, a SP6 promoter, a Lac promoter, etc. The term "terminator" as used herein refers to transcription terminators which define the end of a transcriptional unit (such as a gene) and initiate the process of releasing the newly synthesized RNA from the transcription machinery. Examples of terminators include but are not limited to: a T7 terminator and a rrnB terminator.

## DETAILED DESCRIPTION OF THE PRESENT INVENTION

The invention is defined by the appended claims.

The inventors of this invention have developed, among other things, a multiplex method to measure the activities of nucleic acid modifying enzymes and screen one or more variable elements of the enzymatic reaction. For example, the method can physically link the activity of a nucleic acid modifying enzyme variant to its own encoding DNA/RNA and its DNA/RNA target molecule, and at the same time, molecularly measure both enzymatic activity and inactivity directly for each variant on individual target molecules, regardless of activity levels (i.e. quantifying how active an active variant is, inactive variants can also be measured as 'inactive'). This enables a direct path towards engineering nucleic acid modifying enzymes (e.g. CRISPR-Cas) to have enhanced or novel functionalities (via the active variants), and at the same time builds a fitness landscape map of the sequence variations that are currently non-productive (via the inactive or less active variants). Similarly, variants of guide RNAs and/or DNA/RNA targets can also be screened using the methods disclosed herein.

A non-limiting and non-exhaustive list of the key concepts of this invention are described as follows: (i) a polynucleotide construct that encodes a DNA/RNA target site and a variable element to be tested (for example, a nucleic acid modifying enzyme variant), (ii) mixing the DNA with any of the commonly available RNA and protein-expressing reagents (a.k.a. cell-free transcription-translation (TXTL)/in vitro transcription-translation (IVTT) reaction), (iii) encapsulating or compartmentalizing single copies of DNA construct variants together with IVTT reagents, (iv) allowing IVTT reactions within individual compartments that expresses the nucleic acid modifying enzyme and sgRNA (if the enzyme is an RNA-guided enzyme) in each compartment (and in some embodiments, RNA targets that are co-transcribed as part of the Cas transcript) in isolation from the other multitude of compartments, (v) individual polynucleotide constructs (or in some embodiments, the RNA targets transcribed from the construct) will be cleaved, intact, or otherwise modified depending on the functionality of the encoded nucleic acid modifying enzyme, (vi) quantification of the cleaved, intact, or modified polynucleotide construct (or in some embodiments, the RNA target) in parallel, for example by single molecule long-read sequencing, thereby directly identifying and directly quantifying the enzymatic activity associated with each variable element in a molecularly parallelized manner. This technology links the phenotype of the encoded variable element (e.g. a nucleic acid modifying enzyme variant) to its coding sequence directly, allowing a sequence-function relationship to be determined rapidly for a large library of variants. Figure 1 depicts a non-limiting list of the key concepts of the present invention.

Methods

The methods disclosed herein may be characterized as methods for measuring enzymatic activities. As the methods are highly scalable and are capable of screening large numbers of variant polynucleotides, the method may also be characterized as methods for screening nucleic modifying enzymes, and/or DNA/RNA targets (of the nucleic modifying enzymes), and/or guide RNAs, and/or other components of the enzymatic reaction which can be encoded on the polynucleotide construct. Therefore, in one aspect, the present disclosure refers to a method comprising the steps of:

a) segregating a plurality of polynucleotide constructs into compartments, wherein each compartment comprises a single polynucleotide construct, wherein each polynucleotide construct comprises:

i) a first polynucleotide sequence encoding a nucleic acid modifying enzyme or a variant thereof, operably linked to a first promoter; and
ii) a second polynucleotide sequence comprising a DNA target or a DNA template encoding an RNA target, wherein when the second polynucleotide sequence comprises a DNA template encoding an RNA target, said RNA target is co-expressed contiguously with the nucleic acid modifying enzyme as a single transcript, driven by the first promoter;

and wherein the plurality of the polynucleotide constructs encode different variants of the nucleic acid modifying enzyme, and/or different DNA or RNA targets;
b) subjecting the compartments to conditions which allow *in vitro* expression of RNAs and proteins;
c) subjecting the plurality of the compartments to conditions which allow the modification of DNA/RNA targets by nucleic acid modifying enzymes which have modification activity towards said DNA or RNA targets, thereby producing a population of DNA/RNA molecules that comprises one or more of the following:

iii. polynucleotide constructs and/or RNA targets or fragments thereof that have been modified by the nucleic acid modifying enzyme(s);
iv. polynucleotide constructs and/or RNA targets which have not been modified by the nucleic acid modifying enzyme(s);

d) harvesting the population of DNA/RNA molecules produced in step (c) and subjecting the same to single molecule sequencing;
e) detecting and counting the DNA/RNA molecules referred to in step c)i and c)ii based on the sequencing results.

In this first aspect, the polynucleotide construct encodes both a nucleic acid modifying enzyme (or a variant thereof) and a DNA/RNA target. Accordingly, either the nucleic acid modifying enzyme or the DNA/RNA target may be tested or screened as a variable element. In some examples wherein the method is used for testing or measuring the activity of different nucleic acid modifying enzymes towards a specific target (i.e. screening enzymes), the plurality of the polynucleotide constructs may encode the same DNA/RNA target but different nucleic acid modifying enzymes (or different variants of the same nucleic acid modifying enzyme). In some examples wherein the method is used for testing or measuring the activity of a specific nucleic acid modifying enzyme towards different DNA/RNA targets (i.e. screening DNA/RNA targets), the plurality of the polynucleotide constructs may encode the same nucleic acid modifying enzyme but different DNA/RNA targets. In the context of CRISPR-Cas targets, the expression "different DNA/RNA targets" may refer to DNA/RNA targets which differ in the protospacer (sequence complementary to the guide RNA) or in the PAM/PFS sequence.

In some examples, wherein the nucleic acid modifying enzyme encoded by each polynucleotide construct is an RNA-guided nucleic acid modifying enzyme (such as a CRISPR-Cas nuclease or a variant thereof), a guide RNA (gRNA) may be required for the nucleic acid modifying enzyme to bind and/or modify the DNA/RNA target. In some examples, the gRNA is provided directly to each compartment, either in the form of gRNA or in the form of a DNA template that encodes the gRNA. Therefore in one example, wherein the nucleic acid modifying enzyme is an RNA-guided nucleic acid modifying enzyme, each compartment further comprises a guide RNA or a nucleotide template encoding the same.

In some other examples, the gRNA may be encoded on the same polynucleotide construct which encodes the enzyme and the DNA/RNA target. Therefore in one example, the nucleic acid modifying enzyme is an RNA-guided nucleic acid modifying enzyme, wherein each polynucleotide further comprises a third polynucleotide sequence encoding a variant guide RNA (gRNA); and wherein the plurality of the polynucleotide constructs encode different variants of the nucleic acid modifying enzyme, and/or different DNA or RNA targets, and/or different gRNAs. In this example, the method as disclosed herein comprises the steps of:

a) segregating a plurality of polynucleotide constructs into compartments, wherein each compartment comprises a single polynucleotide construct, wherein each polynucleotide construct comprises:

i) a first polynucleotide sequence encoding a nucleic acid modifying enzyme or a variant thereof, operably linked to a first promoter, wherein the nucleic acid modifying enzyme is an RNA-guided nucleic acid modifying enzyme;
ii) a second polynucleotide sequence comprising a DNA target or a DNA template encoding an RNA target, wherein when the second polynucleotide sequence comprises a DNA template encoding an RNA target, said RNA target is co-expressed contiguously with the nucleic acid modifying enzyme as a single transcript, driven by the first promoter; and
iii) a third polynucleotide sequence encoding a variant guide RNA (gRNA);

wherein the plurality of the polynucleotide constructs encode different variants of the nucleic acid modifying enzyme, and/or different DNA or RNA targets, and/or different gRNAs;
b) subjecting the compartments to conditions which allow *in vitro* expression of RNAs and proteins;
c) subjecting the plurality of the compartments to conditions which allow the modification of DNA/RNA targets by nucleic acid modifying enzymes which have functional activity towards said DNA or RNA targets in the presence of a gRNA, thereby producing a population of DNA/RNA molecules that comprises one or more of the following:

i. polynucleotide constructs and/or RNA targets or fragments thereof that have been modified by the nucleic acid modifying enzyme(s);
ii. polynucleotide constructs and/or RNA targets which have not been modified by the nucleic acid modifying enzyme(s);

d) harvesting the population of DNA/RNA molecules produced in step (c) and subjecting the same to single molecule long-read sequencing;
e) detecting and counting the DNA/RNA molecules referred to in step c)i and c)ii based on the sequencing results.

In the above example, since the gRNA (or the sequence encoding said gRNA) is physically linked to nucleic acid modifying enzyme and the DNA/RNA target, any one of the gRNA, the DNA/RNA target and nucleic acid modifying enzyme may be tested and screened as the variable element. The encoded gRNA is to be expressed from the polynucleotide construct (for example in a compartment), therefore the polynucleotide construct may comprise other elements which facilitate the expression of the gRNA, which will be known generally to a person skilled in the art. In some examples, the third polynucleotide sequence is operably linked to a second promoter. In some examples, the second promoter is a T7 promoter.

Screening nucleic acid modifying enzymes

In some examples wherein the method is used for testing or measuring the activity of different nucleic acid modifying enzymes towards a specific target, the plurality of the polynucleotide constructs may encode the same DNA/RNA target and gRNA, but different nucleic acid modifying enzymes (or different variants of the same nucleic acid modifying enzyme).

One example of nucleic acid modifying enzyme that can be tested, screened and optimized using this method is the Cas family of nucleases. Various CRISPR-Cas systems have been developed in recent years for DNA and RNA editing, enabling a broad spectrum of applications that impact all fields of medicine and biotechnology. Class 2 Cas (CRISPR-associated) proteins, including the Cas9, Cas12 (previously known as Cpf1), Cas13, and Cas14 nucleases that have been well-characterized in the literature, are of particular interest. These Cas proteins are single-component nuclease effectors (i.e. a single Cas protein, not a multimeric complex of different proteins); they typically utilize an RNA oligonucleotide (guide RNA, gRNA; the engineered form also known as single guide RNA, sgRNA; used interchangeably) to program and colocalise the Cas protein to a specific loci on DNA and/or RNA, following which enzymatic activities can occur, such as cleavage (endonucleolytic breakage in the DNA/RNA). A segment of the gRNA sequence (spacer) is complementary to the target sequence of DNA/RNA (protospacer). Another short sequence (typically 2-6nt in length) adjacent to the protospacer, also known as the protospacer adjacent motif (PAM; when on DNA) or protospacer flanking sequence (PFS; when on RNA), is required for functional targeting. Each Cas-gRNA system can recognize unique PAM/PFS sites and have different gRNA:protospacer requirements. Cas proteins have been and can be further engineered to recognize new PAM/PFS sites, have less stringent gRNA lengths or structures, and be more specific and efficient. To use Cas nucleases as therapeutics while minimizing adverse immune responses, immunogenic epitopes in the Cas proteins can also be removed or masked, specifically by deleting or changing the amino acid sequences while maintaining Cas function. New functions can also be engineered into the Cas proteins or Cas fusion proteins, such as to effect base-editing (changing a target nucleotide to another), epigenetic modification, or many other modifications yet to be demonstrated. These efforts usually entail some form of directed evolution, protein engineering, selecting, and screening of Cas variant libraries. The method disclosed herein is useful to measure and screen the activities of large libraries of enzyme (such as Cas) mutants, because it is i) highly scalable, with >$10^9$ compartmentalized IVTT reaction droplets per mL capable of being run in parallel; and ii) compatible with a larger sequence space, which is especially important and useful when working with large proteins (>$10^3$ aa long), such as CRISPR-Cas proteins.

Screening DNA/RNA targets of nucleic acid modifying enzymes

In some examples wherein the method is used for testing or measuring the activity of a specific nucleic acid modifying enzyme towards different DNA/RNA targets in the presence of a specific gRNA, the plurality of the polynucleotide constructs may encode the same nucleic acid modifying enzyme and gRNA, but different DNA/RNA targets.

In these examples, the methods disclosed herein can be used for evaluation of ability of PAM or PFS variants to direct the binding or modification of a DNA/RNA target by an RNA-guided nucleic acid modifying enzyme. The methods disclosed herein allow for the simultaneous assessment of a plurality of PAM/PFS variants for any given target site. Accordingly, data obtained from such methods can be used to compile a list of PAM variants that modify (such as cleaving) a particular DNA/RNA target. It would be readily apparent to a person skilled in the art that any non-PAM/PFS sequences on the target site which may have an effect on the activity of the enzyme may also be tested and screened using this method.

Screening guide RNA

In some examples wherein the method is used for testing or measuring the activity of a specific nucleic acid modifying enzyme towards a specific DNA/RNA target in the presence of different specific gRNAs, the plurality of the polynucleotide constructs may encode the same nucleic acid modifying enzyme and DNA/RNA target, but different gRNAs.

In these examples, the present disclosure provides methods of assessing different gRNAs for ability mediate the binding and/or modification of a nucleic acid modifying enzyme towards a specific DNA/RNA target. Accordingly, results obtained from the methods can be used to compile a list of guide RNA variants that mediate modification of a particular target by a particular nucleic acid modifying enzyme.

In another aspect, the present disclosure refers to a method comprising the steps of:

a) segregating a plurality of polynucleotide constructs into compartments, wherein each compartment comprises a single polynucleotide construct, wherein each polynucleotide construct comprises:

i) a first polynucleotide sequence encoding a guide RNA (gRNA) operably linked to a first promoter;
ii) a second polynucleotide sequence comprising a DNA target or a DNA template encoding an RNA target, wherein when the second polynucleotide sequence comprises a DNA template encoding an RNA target, said RNA target is co-expressed contiguously with the gRNA as a single RNA transcript, driven by the first promoter;

wherein the plurality of the polynucleotide constructs encode different gRNAs, and/or different DNA or RNA targets; and wherein each compartment further comprises an RNA-guided nucleic acid modifying enzyme or a variant thereof or a nucleotide template encoding the same;

b) subjecting the compartments to conditions which allow in vitro transcription and/or translation of RNAs and proteins;
c) subjecting the compartments to conditions which allow the modification of DNA and/or RNA targets by RNA-guided nucleic acid modifying enzymes which have functional activity towards said DNA or RNA targets in the presence of a gRNA, thereby producing a population of DNA/RNA molecules that comprises one or more of the following:

i) polynucleotide constructs and/or RNA transcripts or fragments thereof that have been modified by the nucleic acid modifying enzyme(s);
ii) polynucleotide constructs and/or RNA transcripts which have not been modified by the nucleic acid modifying enzyme(s);

d) harvesting the population of DNA/RNA molecules produced in step (c) and subjecting the same to single molecule long-read sequencing;
e) detecting and counting the DNA/RNA molecules referred to in step c)i and c)ii based on the sequencing results. based on the sequencing results.

In this aspect, the polynucleotide construct encodes a guide RNA (gRNA) and a DNA/RNA target, whereas the RNA guided nucleic acid modifying enzyme is provided to each compartment separately. Accordingly, either the gRNA or the DNA/RNA target may be tested or screened as a variable element. In some examples wherein the method is used for testing or measuring the activity of a specific nucleic acid modifying enzymes towards a specific target in the presence of different gRNAs (i.e. screening gRNAs), the plurality of the polynucleotide constructs may encode the same DNA/RNA target but different nucleic acid modifying enzymes (or different variants of the same nucleic acid modifying enzyme). In some examples wherein the method is used for testing or measuring the activity of a specific nucleic acid modifying enzyme towards different DNA/RNA targets (i.e. screening DNA/RNA targets), the plurality of the polynucleotide constructs may encode the same gRNA but different DNA/RNA targets.

Segregating polynucleotide constructs into compartments

Multiple ways of segregating polynucleotide constructs into compartments are known to the person skilled in the art. In one example, the polynucleotide constructs are segregated into emulsion droplets, by emulsification methods that are commonly known in the art. Generally, emulsions may be produced from any suitable combination of immiscible liquids. In a typical example, emulsions comprise an aqueous phase which encompasses (a) components required for in vitro transcription and translation; and (b) a library of nucleic acid templates described herein. In the emulsion, the aqueous phase is present in the form of finely divided droplets (the disperse, internal or discontinuous phase). The emulsion further comprises a hydrophobic, immiscible liquid (an "oil") as the matrix in which droplets are suspended (the non-disperse, continuous or external phase). Such emulsions are termed "water-in-oil" (W/O), and the droplets are termed "water-in-oil droplets. Many oils and many emulsifiers are known in the art and can be used for the generation of water-in-oil emulsions. Suitable emulsifiers include, e.g., light white mineral oil and surfactants such as sorbitan monooleate (Span80; ICI) and polyoxyethylenesorbitan monooleate (Tween 80; ICI), or any combination thereof. In one example, the emulsifier comprises Mineral Oil, Span 80 and a surfactant, such as Tween 80; such as Mineral Oil + 4.5 % (v/v) Span 80 + 0.5 % (v/v) Tween 80). The testing of different emulsifiers are within the knowledge of the skilled person in the art. In some examples, emulsions are produced using mechanical energy to force the phases together. Various methods can be employed, including, without limitation, use of mechanical devices, including stirrers (such as magnetic stir-bars, propeller and turbine stirrers, paddle devices and whisks), homogenizers (including rotor-stator homogenizers, high-pressure valve homogenizers and jet homogenizers), colloid mills, and ultrasound and "membrane emulsification" devices. The size of emulsion droplets (the compartments) can be varied by those of skill in the art by tailoring the emulsion conditions used to form the emulsion according to requirements of the selection system.

A non-limiting example is described herein: Generating water-in-oil (w/o) emulsion droplets using the following steps or other methods known to persons ordinarily skilled in the art thereof. In summary, add 950 $\mu$L of an oil surfactant mix (Mineral Oil + 4.5 % (v/v) Span 80 + 0.5 % (v/v) Tween 80) to a cryovial with a 3 x 8 mm magnetic stir bar; place on ice. Mix $\leq$1.66 fmol of the DNA library with IVTT reagents (New England Biolabs PURExpress #E6800) on ice to produce a 50 $\mu$L IVTT aqueous mixture. Adding this 50 $\mu$L aqueous mixture in 5 aliquots of 10 $\mu$L over 2 minutes to the oil surfactant mix on ice while the stir bar is spinning at 1150 rpm to generate an emulsion mixture. Allow the emulsion mixture to continue mixing for an additional minute on ice. In one example, a homogenizer (e.g. IKA Ultraturrax T10 homogenizer) is used to mix the stirred emulsion mixture for an additional 3 min at 8000 rpm to achieve a more monodisperse distribution of emulsion droplet diameters.

Other methods of emulsion droplet generation are also possible and would be known to the person skilled in the art, which include vortexing the aqueous and oil mixture, or using a microfluidics device e.g. Dolomite-Bio's $\mu$-encapsulator to control flow rates of aqueous and oil inputs fed into a microfluidic chip junction to encapsulate aqueous solutions with oil in emulsion droplets.

Other compartmentalization methods are also known to persons having ordinary skills in the art. Both virtual and physical compartmentalization are encompassed by the term "compartments" as used herein, as long as the compartmentation enables the segregation of the polynucleotide constructs, reagents and the reactions without creating physical encapsulation. In one example, the segregation of compartments is achieved using microfluidics, hydrogel-limited diffusion, or partitioned wells (or nanowells).

IVTT systems

In some examples of the methods as disclosed herein, each of the compartments comprises *in vitro* transcription and translation (IVTT) reagents, said IVTT reagents enable the *in vitro* transcription and/or translation of proteins and/or RNAs. The inclusion of IVTT in the compartments bypasses the use of cells in the assay. In some embodiments, an IVTT system includes a cell extract, e.g., from bacteria, rabbit reticulocytes or wheat germ. Many suitable systems are commercially available (for example from ThermoFisher, Promega and New England Biolabs). In one example, the system may be emulsified together with the polynucleotide constructs. The conditions suitable for *in vitro* transcription and translation as mentioned in step b) will be apparent or accessible to the person skilled in the arts, either by referring to literature or to manuals of commercial kits. In one non-limiting example, a suitable condition is a 4 h 37 °C incubation. The IVTT reaction may be stopped by methods well known in the art or described in the commercial kit manual. In one example, the compartments comprising the IVTT are incubated at 65 °C for 15 min for heat inactivation of IVTT reagents and any expressed nucleic acid modifying enzyme. In another example, 20 mM EDTA (pH 8.0) inhibitor is added to the compartments (such as emulsion droplets) and mixed.

By controlling the compartmentalization conditions of IVTT reagents with DNA, it is possible to ensure that no more than a single copy of the polynucleotide construct is encapsulated together with IVTT reagents in each compartment, with volumes ranging from femtolitre to nanolitre range. This enables the physical isolation of each variant copy of DNA (and hence the IVTT RNA and protein products) within each compartment, which allows the user to physically confine the expressed RNAs and proteins with their respective encoding DNAs.

Conditions which allow the modification of DNA/RNA targets by known nucleic acid modifying enzymes are generally known in the art and/or can easily be discovered or optimized. For newly discovered enzymes, such conditions can generally be approximated using information about related nucleases that are better characterized (e.g., homologs and orthologs). The modification may refer to any chemical or physical changes to the components or structure of the target, which includes the breaking/cleaving the polynucleotide, creating a nick (single strand breakage) in a double stranded polynucleotide, substituting one or more nucleotide bases, inserting or deleting one or more nucleotide bases, or covalently modifying nucleotide bases with chemical and epigenetic markers (such as cytosine methylation and hydroxymethylation).

As each compartment comprises a single copy of a polynucleotide construct, the DNA/RNA target (which is either comprised on the polynucleotide construct or expressed from said construct) and the nucleic acid modifying enzyme are also confined in said compartment. The activity (or lack thereof) of the nucleic acid modifying enzyme encoded on a specific construct towards the DNA/RNA target encoded on the same construct will manifest in the modification (or lack thereof) of the DNA/RNA target. As the compartments collectively comprise a plurality of different polynucleotide constructs, step c) produces a population of DNA/RNA molecules that comprises one or more of the following:

  i. polynucleotide constructs and/or RNA transcripts or fragments thereof that have been modified by the nucleic acid modifying enzyme(s);
  ii. polynucleotide constructs and/or RNA transcripts which have not been modified by the nucleic acid modifying enzyme(s);

wherein the polynucleotide construct comprises a DNA target, the polynucleotide construct will be modified if the encoded nucleic acid modifying enzyme has activity towards said DNA target. The status of the polynucleotide construct (modified or not modified) is therefore linked to the enzyme by the enzyme-specific sequence comprised on the same construct. Wherein the polynucleotide construct comprises a DNA template encoding an RNA target, the RNA target will be comprised on a transcript RNA expressed from said DNA template. As the RNA target is co-expressed contiguously with the nucleic acid modifying enzyme as a single transcript, the status of the RNA target (modified or not modified) is also linked to the enzyme by the enzyme-specific sequence comprised on the RNA transcript.

Harvesting the DNA/RNA molecules

In order to measure the activity of the nucleic acid modifying enzyme (s) against DNA/RNA target(s), the population of DNA/RNA molecules produced in step c) are harvested before being subjected to sequencing. In some examples, the harvesting of the DNA/RNA molecules requires the breaking of the compartments. Therefore in one example of the method as disclosed herein, step d) further comprises breaking the compartments by physical or chemical methods. In examples wherein the compartments are emulsion droplets, harvesting the DNA/RNA molecules involves the breaking of the emulsion droplets.

Methods of breaking the emulsion droplets are known to persons ordinarily skilled in the art. One non-limiting example of the method is as follows: Transfer the emulsion mixture to a 2 mL centrifuge tube and centrifuge at 13000 g for 5 min at room temperature. Dispose of the upper oil layer. Add 1 mL of water-saturated diethyl ether to the remaining aqueous layer, vortex, and remove the upper solvent layer; repeat this step once. Centrifuge the remaining aqueous layer under vacuum at room temperature for 5 min. In one example, the step of harvesting the DNA/RNA molecules also comprises an IVTT quenching step. The IVTT quenching step can be performed, for example, by treating the remaining aqueous layer with RNase cocktail and Proteinase K to remove excess RNA and proteins from the IVTT reaction. In some examples, the step of harvesting the DNA/RNA molecules also comprises a clean-up step to purify the DNA/RNA molecules. Methods of DNA/RNA clean up are well known to a person of ordinary skills in the art, and there are numerous commercial kits for this process, e.g. DNA Clean & Concentrator-5 (Zymo Research) or SPRIselect bead cleanup (Beckman Coulter).

In some examples, the harvesting of the DNA/RNA molecules requires the purification of the harvested DNA/RNA molecules to remove excess or unwanted DNA, RNA and/or proteins from the reaction. Therefore in one example of the method as disclosed herein, step d) further comprises purifying the harvested DNA/RNA molecules to remove excess DNA, RNA and/or proteins from the reaction. In some examples, excess DNA, RNA and/or proteins may include but are not limited to gRNAs, nucleic acid modifying enzymes, and IVTT reagents. In some examples, the term "excess" describes molecules which are subject to sequencing.

Sequencing

In a preferred example, the sequencing is single molecule sequencing. "Single molecule sequencing" refers to techniques that can read the base sequence directly from individual strands of DNA or RNA present in a sample. At least two types of single molecule sequencing is commercially available: (a) single-molecule sequencing in real-time (SMRT) by Pacific Biosciences, based on fluorophore-labeled nucleotide detection and identification in waveguides smaller than the wavelength (ZMW), and (b) label-free sequencing method that uses an electronic means of reading the signals when threading the nucleic acid (DNA/RNA) fragment through the nanopore used by Oxford Nanopore Technologies. Single molecule sequencing is facilitated by long read lengths and may also be referred to as "long read sequencing" or "single molecule long-read sequencing". The use of single molecule sequencing provides direct identification of variant sequences, which bypasses (i) ligating oligonucleotides to predetermined DNA/RNA ends, and (ii) PCR amplification.

The "direct" detection of enzymatic products of individual variants and the molecular counting of modified:unmodified DNA/RNA targets (or polynucleotide constructs/RNA transcripts) to quantitate the molecular activity of individual variants are an important feature of the present invention. The term "direct" may refer to the direct detection of reaction products, or to the direct measurement of the enzymatic activity of individual variants. In the latter meaning, the expression "direct measurement of enzymatic function" is in the context of directly calculating the phenotypic activity of a variant molecule (in a large scale survey of variants) by the linked genotype information (also encoded within an individual molecule (either the polynucleotide construct or the RNA transcript)). So the enzymatic activity is measured directly on the actual interacting molecules. Based on the methods disclosed herein, the precise level of enzymatic activity can be directly measured based of counts of modified vs unmodified (or total). In an example, a specific variant that is associated with 1:1 modified:unmodified target site is determined to be active on the target site 50% of the time.

Therefore, in some examples, the harvested population of DNA/RNA molecules are not subjected to further modifications before being subjected to the single molecule sequencing reaction, except for modifications required of the single molecule sequencing. These modifications may include those required for conventional sequencing, such as the ligation of cleaved ends to adapters, the attachment of barcodes, the amplification of DNA/RNA molecules by PCR, etc.

In one example, the sequencing is performed using the Oxford Nanopore Technologies platform. A non-limiting example of the sequencing process is described below.

Preparing purified DNA for long-read sequencing following library preparation protocols suggested by the sequencing device manufacturer e.g. Oxford Nanopore Technologies (ONT) MinION Mk1B device and sequence the library accordingly. In some examples, this may involve the use of ONT SQK-LSK109 ligation sequencing kit for general DNA library preparation, together with the ONT EXP-NBD104 PCR-Free native barcoding expansion kit for multiplexing barcoded DNA sublibraries.

Process and analyze the long-read sequencing data using bioinformatics tools on public repositories e.g. minimap2 (Li, H. (2018). Minimap2: pairwise alignment for nucleotide sequences. Bioinformatics, 34:3094-3100. doi:10.1093/bioinformatics/bty191), NanoPack (De Coster, W. et al., (2018). NanoPack: visualizing and processing long-read sequencing data. Bioinformatics, 34:2666-2699. doi: 10.1093/bioinformatics/bty149), samtools (Li, H. et al., (2009). The Sequence Alignment/Map format and SAMtools. Bioinformatics, 25:2078-9. doi: 10.1093/bioinformatics/btp352), VarScan 2 (Koboldt, D.C. et al., (2012). VarScan 2: Somatic mutation and copy number alteration discovery in cancer by exome sequencing. Genome Research, 22: 568-576. doi: 10.1101/gr.129684.111) or custom-made scripts that can be created by a person having ordinary skill in the art of sequencing data analysis. For example, in some examples, the following steps may be taken by a person having ordinary skill in the art of sequencing analysis to process and analyse raw nanopore sequencing reads generated from an ONT sequencing device:

1. Using the ONT-provided guppy toolkit (https://community.nanoporetech.com/protocols/Guppy-protocol/v/gpb_2003_v1_revm_14dec2018), process the raw nanopore sequencing reads with base-calling and de-multiplexing algorithms in the toolkit where necessary. A person having ordinary skill in the art of sequencing analysis may wish to adjust certain parameters such as the filtering threshold for the multiplexed barcode quality score according to their needs. These parameters are customarily described in the respective tool manuals.

2. A person having ordinary skill in the art of sequencing analysis may wish to further filter and process reads based on parameters such as read length and read quality scores using software tools such as NanoPack.

3. These processed reads may then be aligned, using minimap2 or other sequence alignment tools, against a (set of) reference sequence(s) to generate a dataset of read alignments. Likewise, a person having ordinary skill in the art of sequencing analysis may wish to adjust the read alignment parameters such as alignment scoring matrix as needed. These parameters are customarily described in the respective tool manuals.

4. The generated read alignment files can then be parsed by the user to calculate the counts of unmodified and modified reads. In some embodiments, this may occur via the use of other alignment processing tools such as samtools or VarScan2 to detect and identify sequencing variations between the aligned sequencing reads and the reference sequence(s) the sequencing reads were aligned against. Likewise, a person having ordinary skill in the art of sequencing analysis can determine which parameters in these tools should be adjusted as needed e.g. setting the minimum read count threshold to detect and identify true sequencing variations from background levels of sequencing error. These parameters are customarily described in the respective tool manuals.

Molecular detection and counting

The enzymatic activity can thus be directly detected by detecting and counting the polynucleotide constructs and/or RNA transcripts or fragments thereof that have been modified by the nucleic acid modifying enzyme(s), and polynucleotide constructs and/or RNA transcripts which have not been modified by the nucleic acid modifying enzyme(s). The polynucleotide constructs may comprise the DNA target, and the RNA transcripts may comprise the RNA target.

Therefore in some examples, the method further comprises evaluating the modifying activity of one or more nucleic acid modifying enzymes against one or more of the DNA/RNA targets, by calculating the number of polynucleotide constructs and/or RNA transcripts that have been modified by the nucleic acid modifying enzyme ($\Sigma$counts$^{modified}$), and comparing it against the number of polynucleotide constructs and/or RNA transcripts that have not been modified by the nucleic acid modifying enzymes ($\Sigma$counts$^{unmodified}$), or against the total number of polynucleotide constructs and/or RNA transcripts $\Sigma$counts$^{modified}$/$\Sigma$counts$^{unmodified}$.

In one example, the enzymatic activity is represented by a value calculated using any one of the following formulas:

1)

$$enzymatic\ activity \approx \sum counts^{modified} / \sum counts^{unmodified}$$

2)

$$enzymatic\ activity \approx \sum counts^{modified} / \sum counts^{modified+unmodified}.$$

The polynucleotide constructs and/or RNA transcripts or fragments thereof that have or have not been modified by the nucleic acid modifying enzyme(s) can be detected and counted using the sequencing data generated by the sequencing platform available to the person skilled in the art. As DNA/RNA molecules are sequenced directly by single molecule sequencing, in one example the detection and counting of the DNA/RNA molecules which have or have not been modified by the nucleic acid modifying enzyme(s) is based only on data generated during the single molecule sequencing and does not require further modifications or processing of the DNA/RNA molecules.

In one example, wherein the modification activity is cleavage activity, and the detection and calculation of modified and unmodified polynucleotide constructs or RNA targets are achieved by aligning sequencing readings of the DNA/RNA molecules against a reference sequence which contains a window of cleavage sites for the nucleic acid modifying enzyme(s), wherein

i) when the 3' end of a DNA/RNA molecule is mapped to a region 3' downstream of the window of cleavage sites, the DNA/RNA molecule is an unmodified polynucleotide constructs or RNA target;
ii) when the 3' end of a DNA/RNA molecule is mapped to a region within the window of cleavage sites, the DNA/RNA molecule is modified polynucleotide constructs or RNA target;
iii) when the 3' end of a DNA/RNA molecule is mapped to a region 5' upstream of the window of cleavage sites, the DNA/RNA molecule is non-informative and is not used for the measurement of modification activity.

In one example, the sequencing reads are determined by their respective mapped end points (i.e. where the sequencing read ends) to determine whether the end points lie within a small window of expected cleavage sites (grey triangle and dotted line on "Cas reference seq"; Figure 3). A non-limiting example is described as follows, wherein the DNA target site resides 3' of the encoded Cas nuclease variant. By this measure, read alignments (aligned against a reference sequence) with 3' ends that map to the reference sequence at sites 3' downstream of the window of expected Cas cleavage sites are considered uncleaved (in dark grey; Figure 3), while read alignments with 3' ends lying within the window of Cas cleavage sites are considered cleaved (in light grey; Figure 3), and finally reads that do not fulfill either criteria are discarded as non-informative as these cannot be empirically determined if they were cleaved or not (in white; Figure 3). In these examples, each cleaved Cas cleavage site represents one polynucleotide construct/RNA transcript which has been modified. Similarly, each uncleaved Cas cleavage site represents one polynucleotide construct/RNA transcript which has not been modified.

In some examples, the sequencing technology can detect or sense the chemical and sequence identity of the target site to determine whether the target is modified or not by the Cas variant. For example, chemical modifications to nucleotides e.g. methylation can be detected using publicly available bioinformatics tools designed to pick out chemically-modified nucleotides in nanopore sequencing reads (Liu, Q., et al. (2019). Detection of DNA base modifications by deep recurrent neural network on Oxford Nanopore sequencing data. Nat Commun 10(1): 2449, doi: 10.1038/s41467-019-10168-2; Liu, Q., et al. (2019). NanoMod: a computational tool to detect DNA modifications using Nanopore long-read sequencing data. BMC Genomics 20(Suppl 1): 78, doi: 10.1186/s12864-018-5372-8; Rand, A. C., et al. (2017). Mapping DNA methylation with high-throughput nanopore sequencing. Nat Methods 14(4): 411-413, doi: 10.1038/nmeth.4189; Simpson, J. T., et al. (2017). Detecting DNA cytosine methylation using nanopore sequencing. Nat Methods 14(4): 407-410, doi: 10.1038/nmeth.4184). In other examples where the encoded Cas nuclease targets RNA constructs, RNA molecules can be harvested and purified after the IVTT reactions using commercial kits e.g. RNA Clean & Concentrator -5 (Zymo Research), while Oxford Nanopore Technologies provides for the direct nanopore sequencing of harvested RNA molecules with their commercial SQK-RNA002 Direct RNA Sequencing Kit. The sequencing technology can thus be used to detect various types of modifications, including but not limited to strand breaks, sequence-change, and epigenetic biochemical marks.

Polynucleotide constructs and libraries

Figure 2 shows some examples of the polynucleotide constructs as described herein.

In some examples of the method as disclosed herein, the first and second polynucleotide sequences are fully or partially overlapping. For example, the DNA/RNA target ("second polynucleotide") may be encoded within the coding sequence of the nucleic acid modifying enzyme ("first polynucleotide").

In some examples of the method as disclosed herein, the DNA or RNA target comprises a protospacer that is at least partially complementary to the guide RNA. In some examples of the method or the polynucleotide construct as disclosed herein, wherein the DNA target also comprises a proximal Protospacer Adjacent Motif (PAM) sequence. In some examples of the method or the polynucleotide construct as disclosed herein, wherein when the polynucleotide construct comprises a DNA template encoding an RNA target, the RNA target further comprises a proximal Protospacer Flanking Sequence (PFS).

In some examples of the method as disclosed herein, the RNA-guided nucleic acid modifying enzyme is a CRISPR-associated (Cas) protein. In a specific example, the RNA-guided nucleic acid modifying enzyme is selected from the group consisting of a Cas3, a Cas9, a Cas10, a Cas12a (also known as Cpf1), a Cas13a (also known as C2c2), a Cas13b, a Cas13c, a Cas13d, a Cas14, a CasX, a CasΦ, and variants thereof.

In some examples of the method as disclosed herein, the variant nucleic acid modifying enzyme contains one or more inactivated catalytic sites, and is capable of binding and inhibiting the expression of a DNA target, without modifying the DNA target.

In some examples of the method as disclosed herein, the variant nucleic acid modifying enzyme is fused with one or more additional functional domains capable of modifying DNA or RNA. In some specific examples, the additional functional domain(s) include but is not limited to: a Cytidine deaminase domain, a de novo DNA methyltransferase 3A (DNMT3A) domain, a cytosine-5 methyltransferase domain, Ten-Eleven translocation dioxygenase 1 (TET1) catalytic domain, an adenosine deaminases acting on RNA (ADAR2) deaminase domain, and a DNA deoxyadenosine deaminase domain, etc.

For illustrative and exemplary purposes, a sequence of a polynucleotide construct as described herein is provided below.

GCGACATCGTATAACGTTACTGGTTTCACATTCACCACCCTGAATTGACTCTCTTCCGGGCG

CTATCATGCCATACCGCGAAAGGTTTTGCGCCATTCGATGGTGTCCGGGATCTCGACGCTCT

CCCTTATGCGACTCCTGCATTAGGAAAT**TAATACGACTCACTATAGGGGAATTGTGAGCGG**

**ATAACAATTCC**CCTGTAGAAATAATTTTGTTTAACTTTAAT**AAGGAG**ATATACC<u>ATGGACAAG</u>

<u>AAGTACTCCATTGGGCTCGATATCGGCACAAACAGCGTCGGCTGGGCCGTCATTACGGACG</u>

<u>AGTACAAGGTGCCGAGCAAAAAATTCAAAGTTCTGGGCAATACCGATCGCCACAGCATAAAG</u>

<u>AAGAACCTCATTGGCGCCCTCCTGTTCGACTCCGGGGAAACGGCCGAAGCCACGCGGCTC</u>

<u>AAAAGAACAGCACGGCGCAGATATACCCGCAGAAAGAATCGGATCTGCTACCTGCAGGAGA</u>

<u>TCTTTAGTAATGAGATGGCTAAGGTGGATGACTCTTTCTTCCATAGGCTGGAGGAGTCCTTTT</u>

<u>TGGTGGAGGAGGATAAAAAGCACGAGCGCCACCCAATCTTTGGCAATATCGTGGACGAGGT</u>

<u>GGCGTACCATGAAAAGTACCCAACCATATATCATCTGAGGAAGAAGCTTGTAGACAGTACTG</u>

<u>ATAAGGCTGACTTGCGGTTGATCTATCTCGCGCTGGCGCATATGATCAAATTTCGGGGACAC</u>

<u>TTCCTCATCGAGGGGGACCTGAACCCAGACAACAGCGATGTCGACAAACTCTTTATCCAACT</u>

<u>GGTTCAGACTTACAATCAGCTTTTCGAAGAGAACCCGATCAACGCATCCGGAGTTGACGCCA</u>

<u>AAGCAATCCTGAGCGCTAGGCTGTCCAAATCCCGGCGGCTCGAAAACCTCATCGCACAGCT</u>

<u>CCCTGGGGAGAAGAAGAACGGCCTGTTTGGTAATCTTATCGCCCTGTCACTCGGGCTGACC</u>

<u>CCCAACTTTAAATCTAACTTCGACCTGGCCGAAGATGCCAAGCTTCAACTGAGCAAAGACAC</u>

CTACGATGATGATCTCGACAATCTGCTGGCCCAGATCGGCGACCAGTACGCAGACCTTTTTT
TGGCGGCAAAGAACCTGTCAGACGCCATTCTGCTGAGTGATATTCTGCGAGTGAACACGGA
GATCACCAAAGCTCCGCTGAGCGCTAGTATGATCAAGCGCTATGATGAGCACCACCAAGAC
TTGACTTTGCTGAAGGCCCTTGTCAGACAGCAACTGCCTGAGAAGTACAAGGAAATTTTCTT
CGATCAGTCTAAAAATGGCTACGCCGGATACATTGACGGCGGAGCAAGCCAGGAGGAATTT
TACAAATTTATTAAGCCCATCTTGGAAAAAATGGACGGCACCGAGGAGCTGCTGGTAAAGCT
TAACAGAGAAGATCTGTTGCGCAAACAGCGCACTTTCGACAATGGAAGCATCCCCCACCAG
ATTCACCTGGGCGAACTGCACGCTATCCTCAGGCGGCAAGAGGATTTCTACCCCTTTTTGAA
AGATAACAGGGAAAAGATTGAGAAATCCTCACATTTCGGATACCCTACTATGTAGGCCCCC
TCGCCCGGGGAAATTCCAGATTCGCGTGGATGACTCGCAAATCAGAAGAGACCATCACTCC
CTGGAACTTCGAGGAAGTCGTGGATAAGGGGGCCTCTGCCCAGTCCTTCATCGAAAGGATG
ACTAACTTTGATAAAAATCTGCCTAACGAAAGGTGCTTCCTAAACACTCTCTGCTGTACGAG
TACTTCACAGTTTATAACGAGCTCACCAAGGTCAAATACGTCACAGAAGGGATGAGAAAGCC
AGCATTCCTGTCTGGAGAGCAGAAGAAAGCTATCGTGGACCTCCTCTTCAAGACGAACCGG
AAAGTTACCGTGAAACAGCTCAAAGAAGACTATTTCAAAAAGATTGAATGTTTCGACTCTGTT
GAAATCAGCGGAGTGGAGGATCGCTTCAACGCATCCCTGGGAACGTATCACGATCTCCTGA
AAATCATTAAAGACAAGGACTTCCTGGACAATGAGGAGAACGAGGACATTCTTGAGGACATT
GTCCTCACCCTTACGTTGTTTGAAGATAGGGAGATGATTGAAGAACGCTTGAAAACTTACGC
TCATCTCTTCGACGACAAAGTCATGAAACAGCTCAAGAGGCGCCGATATACAGGATGGGGG
CGGCTGTCAAGAAACTGATCAATGGGATCCGAGACAAGCAGAGTGGAAAGACAATCCTGG
ATTTTCTTAAGTCCGATGGATTTGCCAACCGGAACTTCATGCAGTTGATCCATGATGACTCTC
TCACCTTTAAGGAGGACATCCAGAAAGCACAAGTTTCTGGCCAGGGGGACAGTCTTCACGA
GCACATCGCTAATCTTGCAGGTAGCCCAGCTATCAAAAAGGGAATACTGCAGACCGTTAAGG
TCGTGGATGAACTCGTCAAAGTAATGGGAAGGCATAAGCCCGAGAATATCGTTATCGAGATG
GCCCGAGAGAACCAAACTACCCAGAAGGGACAGAAGAACAGTAGGGAAAGGATGAAGAGG
ATTGAAGAGGGTATAAAAGAACTGGGGTCCCAAATCCTTAAGGAACACCCAGTTGAAAACAC
CCAGCTTCAGAATGAGAAGCTCTACCTGTACTACCTGCAGAACGGCAGGGACATGTACGTG
GATCAGGAACTGGACATCAATCGGCTCTCCGACTACGACGTGGATCATATCGTGCCCCAGT
CTTTTCTCAAAGATGATTCTATTGATAATAAAGTGTTGACAAGATCCGATAAAAATAGAGGGA
AGAGTGATAACGTCCCCTCAGAAGAAGTTGTCAAGAAAATGAAAAATTATTGGCGGCAGCTG
CTGAACGCCAAACTGATCACACAACGGAAGTTCGATAATCTGACTAAGGCTGAACGAGGTG
GCCTGTCTGAGTTGGATAAAGCCGGCTTCATCAAAAGGCAGCTTGTTGAGACACGCCAGAT
CACCAAGCACGTGGCCCAAATTCTCGATTCACGCATGAACACCAAGTACGATGAAAATGACA
AACTGATTCGAGAGGTGAAAGTTATTACTCTGAAGTCTAAGCTGGTCTCAGATTTCAGAAAG
GACTTTCAGTTTTATAAGGTGAGAGAGATCAACAATTACCACCATGCGCATGATGCCTACCT
GAATGCAGTGGTAGGCACTGCACTTATCAAAAAATATCCCAAGCTTGAATCTGAATTTGTTTA
CGGAGACTATAAAGTGTACGATGTTAGGAAAATGATCGCAAGTCTGAGCAGGAAATAGGCA
AGGCCACCGCTAAGTACTTCTTTTACAGCAATATTATGAATTTTTTCAAGACCGAGATTACAC

TGGCCAATGGAGAGATTCGGAAGCGACCACTTATCGAAACAAACGGAGAAACAGGAGAAAT
CGTGTGGGACAAGGGTAGGGATTTCGCGACAGTCCGGAAGGTCCTGTCCATGCCGCAGGT
GAACATCGTTAAAAAGACCGAAGTACAGACCGGAGGCTTCTCCAAGGAAAGTATCCTCCCG
AAAAGGAACAGCGACAAGCTGATCGCACGCAAAAAGATTGGGACCCCAAGAAATACGGCG
GATTCGATTCTCCTACAGTCGCTTACAGTGTACTGGTTGTGGCCAAAGTGGAGAAAGGGAAG
TCTAAAAAACTCAAAAGCGTCAAGGAACTGCTGGGCATCACAATCATGGAGCGATCAAGCTT
CGAAAAAACCCCATCGACTTTCTCGAGGCGAAAGGATATAAAGAGGTCAAAAAAGACCTCA
TCATTAAGCTTCCCAAGTACTCTCTCTTTGAGCTTGAAAACGGCCGGAAACGAATGCTCGCT
AGTGCGGGCGAGCTGCAGAAAGGTAACGAGCTGGCACTGCCCTCTAAATACGTTAATTTCTT
GTATCTGGCCAGCCACTATGAAAAGCTCAAAGGGTCTCCCGAAGATAATGAGCAGAAGCAG
CTGTTCGTGGAACAACACAAACACTACCTTGATGAGATCATCGAGCAAATAAGCGAATTCTC
CAAAAGAGTGATCCTCGCCGACGCTAACCTCGATAAGGTGCTTTCTGCTTACAATAAGCACA
GGGATAAGCCCATCAGGGAGCAGGCAGAAACATTATCCACTTGTTTACTCTGACCAACTTG
GGCGCGCCTGCAGCCTTCAAGTACTTCGACACCACCATAGACAGAAAGCGGTACACCTCTA
CAAAGGAGGTCCTGGACGCCACACTGATTCATCAGTCAATTACGGGGCTCTATGAAACAAGA
ATCGACCTCTCTCAGCTCGGTGGAGACAGCAGGGCTGACCCCAAGAAGAAGAGGAAGGTG
GATCCAAAAAAGAAGAGGAAGGTAGATCCCAAGAAAAAAAGAAAAGTCTCGAGCGACTACAA
AGACCATGACGGTGATTATAAAGATCATGACATCGATTACAAGGATGACGATGACAAGTGAG
CTTT**CTAACTAAAAAGGCCTCCCAAATCGGGGGGCCTTTTTTATTGATAACAAAA**CGCTAG
CGGCCGCATAATGCTTAAGTCGAACAGAAAGTAATCGTATTGTACACGGCCGCATAATCGAA
AT**TAATACGACTCACTATAG**GTCTGACAGCAGACGTGCACTGGCCAGGTTTTAGAGCTAGA
**AATAGCAAGTTAAAATAAGGCTAGTCCGTTATCAACTTGAAAAAGTGGCACCGAGTCGGT**
**GCT**CCGCTGAGCAATAA**CTAGCATAACCCCTTGGGGCCTCTAAACGGGTCTTGAGGGGTTT**
**TTTG**TTACCC**TTTATCTGACAGCAGACGTGCACTGGCCAGGGGGAT**GGTTTGGGCCTCACG
TGACATGTGAGCAAAAGCTGAAACCTCAGGCATTTGAGAAGCACACGGTCACACTGCTTCC
GGTAGTCAATAAACCGGTAAACCAGCAATAGACATAAGCGGCTATTTAACGACCCTGCCCTG
AACCGACGACCGGGTCGAATTTGCTTTCGAATTTCTGCCATTCATCCGCTTATTATCACTTAT
TCAGGCGTAGCAACCAGGCGTTTAAGGGCACCAATAACTGCCTTAAAAAAATTACGCCCCGC
CCTGCCACTCATCGCAGTACTGTTGTAATTCATTAAGCATTCTGCCGACATGGAAGCCATCA
CAAACGGCATGATGAACCTGAATCGCCAGCGGCATCAGCACCTTGTCGCCTTGCGTATAATA
TTTGCCCATAGTGAAAACGGGGGCGAAGAAGTTGTCCATATTGGCCACGTTTAAATCAAAC
TGGTGAAACTCACCCAGGGATTGGCTGAGACGAAAACATATTCTCAATAAACCCTTTAGGG
AAATAGGCCAGGTTTTCACCGTAACACGCCACATCTTGCGAATATATGTGTAGAAACTGCCG
GAAATCGTCGTGGTATTCACTCCAGAGCGATGAAACGTTTCAGTTTGCTCATGGAAAACGG
TGTAACAAGGGTGAACACTATCCCATATCACCAGCTCACCGTCTTTCATTGCCATACGGAAC
TCCGGATGAGCATTCATCAGGCGGGCAAGAATGTGAATAAAGGCCGGATAAAACTTGTGCTT
ATTTTTCTTTACGGTCTTTAAAAAGGCCGTAATATCCAGCTGAACGGTCTGGTTATAGGTACA
TTGAGCAACTGACTGAAATGCCTCAAAATGTTCTTTACGATGCCATTGGGATATATCAACGGT

GGTATATCCAGTGATTTTTTTCTCCATTTTAGCTTCCTTAGCTCCTGAAAATCTCGATAACTCA

AAAAATACGCCCGGTAGTGATCTTATTTCATTATGGTGAAAGTTGGAACCTCTTACGTGCCGA

TCAACGTCTCATTTTCGCCAAAAGTTGGCCCAGGGCTTCCCGGTATCAACAGGGACACCAG

GATTTATTTATTCTGCGAAGTGATCTTCCGTCACAGGTATTTATTCGGCGCAAAGTGCGTCG

GGTGATGCTGCCAACTTACTGATTTAGTGTATGATGGTGTTTTTGAGGTGCTCCAGTGGCTT

CTGTTTCTATCAGCTGTCCCTCCTGTTCAGCTACTGACGGGGTGGTGCGTAACGGCAAAAG

CACCGCCGGACATCAGCGCTAGCGGAGTGTATACTGGCTTACTATGTTGGCACTGATGAGG

GTGTCAGTGAAGTGCTTCATGTGGCAGGAGAAAAAAGGCTGCACCGGTGCGTCAGCAGAAT

ATGTGATACAGGATATATTCCGCTTCCTCGCTCACTGACTCGCTACGCTCGGTCGTTCGACT

GCGGCGAGCGGAAATGGCTTACGAACGGGGCGGAGATTTCCTGGAAGATGCCAGGAAGAT

ACTTAACAGGGAAGTGAGAGGGCCGCGGCAAAGCCGTTTTTCCATAGGCTCCGCCCCCCTG

AC (**SEQ ID NO: 10**).

The bold and underlined sequences refer to elements which will be annotated separately below:

**T7/lacO promoter:**
TAATACGACTCACTATAGGGGAATTGTGAGCGGATAACAATTCC **(SEQ ID NO: 1)**

**RBS (Ribosome binding site):** AAGGAG **(SEQ ID NO: 2)**

**Sp Cas9 gene (coding sequence):**

ATGGACAAGAAGTACTCCATTGGGCTCGATATCGGCACAAACAGCGTCGGCTGGGCCGTCA

TTACGGACGAGTACAAGGTGCCGAGCAAAAAATTCAAAGTTCTGGGCAATACCGATCGCCA

CAGCATAAAGAAGAACCTCATTGGCGCCCTCCTGTTCGACTCCGGGGAaACGGCCGAAGCC

ACGCGGCTCAAAAGAACAGCACGGCGCAGATATACCCGCAGAAAGAATCGGATCTGCTACC

TGCAGGAGATCTTTAGTAATGAGATGGCTAAGGTGGATGACTCTTTCTTCCATAGGCTGGAG

GAGTCCTTTTTGGTGGAGGAGGATAAAAAGCACGAGCGCCACCCAATCTTTGGCAATATCGT

GGACGAGGTGGCGTACCATGAAAAGTACCCAACCATATCATCTGAGGAAGAAGCTTGTA

GACAGTACTGATAAGGCTGACTTGCGGTTGATCTATCTCGCGCTGGCGCATATGATCAAATT

TCGGGGACACTTCCTCATCGAGGGGGACCTGAACCCAGACAACAGCGATGTCGACAAACTC

TTTATCCAACTGGTTCAGACTTACAATCAGCTTTTCGAAGAGAACCCGATCAACGCATCCGG

AGTTGACGCCAAGCAATCCTGAGCGCTAGGCTGTCCAAATCCCGGCGGCTCGAAAACCTC

ATCGCACAGCTCCCTGGGGAGAAGAAGAACGGCCTGTTTGGTAATCTTATCGCCCTGTCAC

TCGGGCTGACCCCCAACTTTAAATCTAACTTCGACCTGGCCGAAGATGCCAAGCTTCAACTG

AGCAAGACACCTACGATGATGATCTCGACAATCTGCTGGCCCAGATCGGCGACCAGTACG

CAGACCTTTTTTTGGCGGCAAAGAACCTGTCAGACGCCATTCTGCTGAGTGATATTCTGCGA

GTGAACACGGAGATCACCAAAGCTCCGCTGAGCGCTAGTATGATCAAGCGCTATGATGAGC

ACCACCAAGACTTGACTTTGCTGAAGGCCCTTGTCAGACAGCAACTGCCTGAGAAGTACAAG

GAAATTTTCTTCGATCAGTCTAAAAATGGCTACGCCGGATACATTGACGGCGGAGCAAGCCA

GGAGGAATTTTACAAATTTATTAAGCCCATCTTGGAAAAAATGGACGGCACCGAGGAGCTGC
TGGTAAAGCTTAACAGAGAAGATCTGTTGCGCAAACAGCGCACTTTCGACAATGGAAGCATC
CCCCACCAGATTCACCTGGGCGAACTGCACGCTATCCTCAGGCGGCAAGAGGATTTCTACC
CCTTTTTGAAAGATAACAGGGAAAGATTGAGAAAATCCTCACATTTCGGATACCCTACTATG
TAGGCCCCCTCGCCCGGGGAAATTCCAGATTCGCGTGGATGACTCGCAAATCAGAAGAgAC
CATCACTCCCTGGAACTTCGAGGAAGTCGTGGATAAGGGGGCCTCTGCCCAGTCCTTCATC
GAAAGGATGACTAACTTTGATAAAAATCTGCCTAACGAAAGGTGCTTCCTAAACACTCTCTG
CTGTACGAGTACTTCACAGTTTATAACGAGCTCACCAAGGTCAAATACGTCACAGAAGGGAT
GAGAAAGCCAGCATTCCTGTCTGGAGAGCAGAAGAAAGCTATCGTGGACCTCCTCTTCAAG
ACGAACCGGAAAGTTACCGTGAAACAGCTCAAAGAAGACTATTTCAAAAAGATTGAATGTTTC
GACTCTGTTGAAATCAGCGGAGTGGAGGATCGCTTCAACGCATCCCTGGGAACGTATCACG
ATCTCCTGAAAATCATTAAAGACAAGGACTTCCTGGACAATGAGGAGAACGAGGACATTCTT
GAGGACATTGTCCTCACCCTTACGTTGTTTGAAGATAGGGAGATGATTGAAGAACGCTTGAA
AACTTACGCTCATCTCTTCGACGACAAAGTCATGAAACAGCTCAAGAGGCGCCGATATACAG
GATGGGGGCGGCTGTCAAGAAACTGATCAATGGGATCCGAGACAAGCAGAGTGGAAAGA
CAATCCTGGATTTTCTTAAGTCCGATGGATTTGCCAACCGGAACTTCATGCAGTTGATCCATG
ATGACTCTCTCACCTTTAAGGAGGACATCCAGAAAGCACAAGTTTCTGGCCAGGGGGACAG
TCTTCACGAGCACATCGCTAATCTTGCAGGTAGCCCAGCTATCAAAAAGGGAATACTGCAGA
CCGTTAAGGTCGTGGATGAACTCGTCAAAGTAATGGGAAGGCATAAGCCCGAGAATATCGTT
ATCGAGATGGCCCGAGAGAACCAAACTACCCAGAAGGGACAGAAGAACAGTAGGGAAAGG
ATGAAGAGGATTGAAGAGGGTATAAAAGAACTGGGGTCCCAAATCCTTAAGGAACACCCAGT
TGAAAACACCCAGCTTCAGAATGAGAAGCTCTACCTGTACTACCTGCAGAACGGCAGGGAC
ATGTACGTGGATCAGGAACTGGACATCAATCGGCTCTCCGACTACGACGTGGATCATATCGT
GCCCCAGTCTTTTCTCAAAGATGATTCTATTGATAATAAAGTGTTGACAAGATCCGATAAAAA
TAGAGGGAAGAGTGATAACGTCCCCTCAGAAGAAGTTGTCAAGAAAATGAAAAATTATTGGC
GGCAGCTGCTGAACGCCAAACTGATCACACAACGGAAGTTCGATAATCTGACTAAGGCTGA
ACGAGGTGGCCTGTCTGAGTTGGATAAAGCCGGCTTCATCAAAAGGCAGCTTGTTGAGACA
CGCCAGATCACCAAGCACGTGGCCCAAATTCTCGATTCACGCATGAACACCAAGTACGATG
AAAATGACAAACTGATTCGAGAGGTGAAAGTTATTACTCTGAAGTCTAAGCTGGTcTCAGATT
TCAGAAAGGACTTTCAGTTTTATAAGGTGAGAGAGATCAACAATTACCACCATGCGCATGAT
GCCTACCTGAATGCAGTGGTAGGCACTGCACTTATCAAAAAATATCCCAAGCTTGAATCTGA
ATTTGTTTACGGAGACTATAAAGTGTACGATGTTAGGAAAATGATCGCAAGTCTGAGCAGG
AAATAGGCAAGGCCACCGCTAAGTACTTCTTTTACAGCAATATTATGAATTTTTTCAAGACCG
AGATTACACTGGCCAATGGAGAGATTCGGAAGCGACCACTTATCGAAACAAACGGAGAAAC
AGGAGAAATCGTGTGGGACAAGGGTAGGGATTTCGCGACAGTCCGGAAGGTCCTGTCCATG
CCGCAGGTGAACATCGTTAAAAAGACCGAAGTACAGACCGGAGGCTTCTCCAAGGAAAGTA
TCCTCCCGAAAAGGAACAGCGACAAGCTGATCGCACGCAAAAAGATTGGGACCCCAAGAA
ATACGGCGGATTCGATTCTCCTACAGTCGCTTACAGTGTACTGGTTGTGGCCAAAGTGGAGA

AAGGGAAGTCTAAAAAACTCAAAAGCGTCAAGGAACTGCTGGGCATCACAATCATGGAGCG
ATCAAGCTTCGAAAAAAACCCCATCGACTTTCTCGAGGCGAAAGGATATAAAGAGGTCAAAA
AAGACCTCATCATTAAGCTTCCCAAGTACTCTCTCTTTGAGCTTGAAAACGGCCGGAAACGA
ATGCTCGCTAGTGCGGGCGAGCTGCAGAAAGGTAACGAGCTGGCACTGCCCTCTAAATACG
TTAATTTCTTGTATCTGGCCAGCCACTATGAAAGCTCAAAGGGTCtCCCGAAGATAATGAGC
AGAAGCAGCTGTTCGTGGAACAACACAAACACTACCTTGATGAGATCATCGAGCAAATAAGC
GAATTCTCCAAAAGAGTGATCCTCGCCGACGCTAACCTCGATAAGGTGCTTTCTGCTTACAA
TAAGCACAGGGATAAGCCCATCAGGGAGCAGGCAGAAAACATTATCCACTTGTTTACTCTGA
CCAACTTGGGCGCGCCTGCAGCCTTCAAGTACTTCGACACCACCATAGACAGAAAGCGGTA
CACCTCTACAAAGGAGGTCCTGGACGCCACACTGATTCATCAGTCAATTACGGGGCTCTATG
AAACAAGAATCGACCTCTCTCAGCTCGGTGGAGACAGCAGGGCTGACCCCAAGAAGAAGAG
GAAGGTG (**SEQ ID NO: 3**)

**Synthetic terminator sequence** (L3S1 P52):
TCTAACTAAAAAGGCCTCCCAAATCGGGGGGCCTTTTTTATTGATAACAAAA **(SEQ ID NO: 4)**

**T7 promoter:** TAATACGACTCACTATAG **(SEQ ID NO: 5)**

**gRNA target sequence (protospacer):** TCTGACAGCAGACGTGCACTGGCCAG **(SEQ ID NO: 6)**

**SpCas9 gRNA scaffold:**

GTTTTAGAGCTAGAAATAGCAAGTTAAAATAAGGCTAGTCCGTTATCAACTTGAAAAAGTGGC
ACCGAGTCGGTGCT (**SEQ ID NO: 7**)

**T7 terminator:** CTAGCATAACCCCTTGGGGCCTCTAAACGGGTCTTGAGGGGTTTTTTG **(SEQ ID NO:** 8)

**Target region (a DNA target):** TTTATCTGACAGCAGACGTGCACTGGCCAGGGGGAT **(SEQ ID NO: 9)**

In some examples such as the one exemplified above, a Protospacer Adjacent Motif (PAM) is found next to the target sequence (aka protospacer), for example: 5' PAM site TTTV **(SEQ ID NO: 11)** for Cpf1-type (also known as Cas12) Cas proteins, 3' PAM site NGG for Sp Cas9 and 3' PAM site NNGRRT **(SEQ ID NO: 12)** for Sa Cas9 proteins, flanking the TCTGACAGCAGACGTGCACTGGCCAG **(SEQ ID NO: 6)** protospacer sequence. Standard IUPAC nucleic acid notation is used herein and across the specification.

**EXAMPLES**

**Example 1: IVTT and cleavage of SpCas9 constructs in emulsion droplets**

Water-in-oil (w/o) emulsion droplets were generated following the steps outlined in the protocol provided above. In summary, 950 $\mu$L of an oil surfactant mix (Mineral Oil + 4.5 % (v/v) Span 80 + 0.5 % (v/v) Tween 80) was added to a cryovial with a 3 x 8 mm magnetic stir bar and placed on ice.
*High DNA input: >1 sequence copy encapsulated per emulsion droplet - Demonstrating that emulsification maintains Cas activity as per Cas expressed from bulk IVTT reactions.*
For this experiment, ~ 750 ng of Sp Cas9 construct (sequence as depicted above) with IVTT reagents (New England Biolabs PURExpress #E6800) on ice to produce a 75 $\mu$L IVTT aqueous mixture. 50 $\mu$L of the aqueous mixture was added in 5 aliquots of 10 $\mu$L over 2 minutes to the oil surfactant mix on ice while the stir bar was spinning at 1150 rpm to generate an emulsion mixture. The emulsion mixture was allowed to continue mixing for an additional minute on ice. The emulsion mixture was then subjected to homogenization (8000 rpm for 3 minutes; IKA Ultraturrax T10 homogenizer) to create a more monodisperse distribution of emulsion droplet sizes. The remaining 25 $\mu$L of the aqueous mixture was kept on ice for a bulk IVTT reaction as a control. This was repeated for a Sp dCas9 construct as well.

The emulsion and bulk IVTT mixtures were then incubated for 4 h at 37 °C for IVTT to proceed, followed by 65 °C for 15 min to inactivate the proteins.

The emulsion IVTT mixture was then treated as described above to break the emulsions. 20 mM EDTA (pH 8.0) inhibitor was added to the emulsion and mixed briefly via vortexing. The emulsion mixtures were then centrifuged at 13000 g for 5 min at room temperature. The upper oil layer was removed. 1 mL of water-saturated diethyl ether was added to the remaining aqueous layer, vortexed, and the upper solvent layer was removed; this step was repeated once. The remaining aqueous layer was centrifuged under vacuum at room temperature for 5 min, then treated with RNase cocktail and Proteinase K to remove excess RNA and proteins from the IVTT reaction at 37 °C for 30 min. The bulk IVTT reaction was treated with 20 mM EDTA (pH 8.0) and a mixture of RNase cocktail and Proteinase K to remove excess RNA and proteins from the IVTT reaction at 37 °C for 30 min as well. The DNA from all the IVTT reactions were then purified individually with SPRIselect paramagnetic beads and aliquots were visualized on an agarose gel after size separation via gel electrophoresis (Figure 4). In the emulsion IVTT reactions, constructs encoding active Sp Cas9 were cleaved (presence of smaller band) while constructs encoding inactive Sp dCas9 were uncleaved (absence of smaller band). This demonstrates that the CRISPR-Cas IVTT self-cleaving assay works whether the encoding DNA constructs are compartmentalized in emulsion droplets or free-floating in bulk solutions.

The purified DNA from the emulsion IVTT reactions were also treated for nanopore sequencing using the commercially available SQK-LSK109 ligation sequencing kit from Oxford Nanopore Technologies (ONT), and barcoded using ONT EXP-NBD104 PCR-Free native barcoding expansion kit so they could be optionally multiplexed in a single pooled DNA library. Single-molecule long-read nanopore sequencing was then performed on the pooled DNA library using the ONT MinION Mk1B sequencing device. The nanopore sequencing results were then filtered for quality and analyzed using publicly available bioinformatics tools. The Sp Cas9 emulsion IVTT nanopore sequencing reads show a mix of cleaved and uncleaved construct fragments detected (Figure 5). The Sp dCas9 emulsion IVTT nanopore sequencing reads show up overwhelmingly as uncleaved construct fragments as expected (Figure 6); the tiny minority of reads classified as "cleaved" Sp dCas9 construct fragments are likely the result of truncated/incomplete reads during nanopore sequencing and/or random DNA shearing events and/or errors on the sequencing device. Some reads in each sublibrary were mapped to the wrong sequences e.g. reads mapping to Sp dCas9 instead of Sp Cas9 for a Sp Cas9-only sublibrary. These were likely to be a result of random sequencing error on the sequencing device, or were mis-assigned to their respective sub-libraries during the de-multiplexing of barcoded nanopore sequencing reads; these were thus classified as mis-assigned and depicted as such in the plots.

**Example 2: Quantifying cleavage activities of CRISPR-Cas through multiplex single-molecule long-read sequencing of DNA constructs after bulk IVTT reactions**

Bulk IVTT reactions were set up on ice for different CRISPR-Cas constructs (Sp Cas9, Sa Cas9, As Cpf1, Lb Cpf1) which all shared a similar arrangement of components as described in the nucleic acid template sequence above. These were then divided equally into 5 corresponding aliquots for each time point (Figure 7 Part 1). These bulk IVTT aliquots were then incubated at 37 °C and removed per designated timepoint to be quenched with EDTA inhibitor and enzymes to stop the IVTT reactions and Cas cleavage of encoding DNA constructs (Figure 7 Part 2). The quenched IVTT reactions were then processed with SPRIselect beads cleanup to purify the DNA fragments (Figure 7 Part 3).

Small aliquots of these DNA fragments of the different Cas orthologs from different IVTT timepoints were then visualized on an agarose gel after size separation via gel electrophoresis, as seen in Figure 8.

The remaining aliquots of purified DNA fragments were then pooled by their respective timepoints but irrespective of Cas species i.e. DNA fragments for Sp Cas9, Sa Cas9 etc. at each timepoint were mixed together and were barcoded individually using the ONT EXP-NBD104 PCR-Free native barcoding expansion kit (Figure 7 Part 4) to multiplex these pooled sublibraries for a single nanopore sequencing run (Figure 7 Part 5). The nanopore sequencing results were then filtered for quality and analyzed using publicly available bioinformatics tools, followed by the analytic approach disclosed in this invention.

Figure 9 depicts the counts of cleaved DNA fragments encoding each active Cas constructs normalized against the total counts of cleaved and uncleaved DNA fragments encoding the respective Cas constructs over the selected 5 timepoints of IVTT incubation (between 0 to 4 h). As IVTT incubation duration increases, the expressed Cas proteins have more time to cleave more encoding DNA constructs, resulting in a higher occurrence rate of cleaved fragments for each species at the later timepoints. The nanopore sequencing analysis results plotted in Figure 9 show qualitative agreement with the gel image of purified IVTT DNA fragments in Figure 8, with both assays sharing the same purified DNA input that was obtained from the workflow step depicted in Figure 7 Part 3. This example demonstrates that our claims of surveying nucleic acid products from individual IVTT reactions of multiple CRISPR-Cas self-cleaving assays in our workflow.

**Example** 3: **Demonstration of nanopore-sequencing assay sensitivity by titrating ratios of purified CRISPR-Cas DNA end-products from bulk IVTT reactions**

For this experiment, 500 ng of Sp Cas9 (sequence as depicted in above) with IVTT reagents (New England Biolabs PURExpress #E6800) on ice to produce a 50 μL IVTT aqueous mixture. The same was done for a Sp dCas9 construct as well; the Sp dCas9 construct contains an essentially identical DNA sequence to that of the Sp Cas9 construct, except for 2 deactivating mutations in the Sp Cas9 gene (D10A and H840A) to yield a Sp dCas9 gene. These 50 μL bulk IVTT reactions were incubated at 37 °C for 4 h for IVTT to proceed, followed by 65 °C for 15 min to inactivate the proteins. 20 mM EDTA (pH 8.0) inhibitor with RNase cocktail and Proteinase K were added to the bulk IVTT reactions to remove excess RNA and proteins from the IVTT reaction at 37 °C for 30 min. The DNA from both bulk IVTT reactions were then purified individually with SPRIselect paramagnetic beads, aliquots of which were then visualized on an agarose gel after size separation via gel electrophoresis, as seen in Figure 10.

The concentrations of the purified DNA from the Sp dCas9 and Sp Cas9 bulk IVTT reactions were quantified then mixed in the following mass ratios: 1:1, $1:10^{-1}$, $1:10^{-2}$, $1:10^{-3}$, $1:10^{-4}$, $1:10^{-5}$, 1:0. These 7 mixtures with ratios of titrated purified Sp dCas9 and Sp Cas9 bulk IVTT DNA products were then treated for nanopore sequencing using the ONT SQK-LSK109 ligation sequencing kit, while each of the 7 mixtures were barcoded individually using the ONT EXP-NBD104 PCR-Free native barcoding expansion kit. Single-molecule long-read nanopore sequencing was then performed on the DNA libraries using the ONT MinION Mk1B sequencing device. The nanopore sequencing results were then filtered for quality and analyzed using publicly available bioinformatics tools, followed by the analytic approach disclosed in this invention.

The purpose of this assay was to assess the sensitivity of the nanopore sequencing assay used for a large-scale survey of DNA/RNA modification events, a capability claimed in our invention. Specifically in this instance, the self-cleavage events of Sp Cas9 IVTT constructs titrated against non-cleavage of Sp dCas9 IVTT constructs. Using a combination of the abovementioned bioinformatics approaches, the inventors demonstrated the detection of cleaved and uncleaved Sp Cas9 DNA fragments that could be distinguished from the detection of the uncleaved Sp dCas9 DNA fragments in the raw nanopore sequencing data. Notably, the inventors were able to detect the presence of cleaved Sp Cas9 DNA fragments even in the $1:10^{-5}$ mix of purified Sp dCas9 and Sp Cas9 bulk IVTT DNA products respectively (Figure 11).

**Example 4: IVTT and cleavage of SpCas9 constructs in emulsion droplets**

Limiting DNA input: ≤1 sequence copy encapsulated per emulsion droplet - Measuring efficiency of emulsifying single copies of DNA construct.

For this experiment, ≤1.66 fmol of Sp Cas9 construct (sequence as depicted above) with IVTT reagents (New England Biolabs PURExpress #E6800) on ice to produce a 50 μL IVTT aqueous mixture. The 50 μL aqueous mixture was added in 5 aliquots of 10 μL over 2 minutes to the oil surfactant mix on ice while the stir bar was spinning at 1150 rpm to generate an emulsion mixture. The emulsion mixture was allowed to continue mixing for an additional minute on ice. The emulsion mixture was then subjected to homogenization (8000 rpm for 3 minutes; IKA Ultraturrax T10 homogenizer) to create a more monodisperse distribution of emulsion droplet sizes. This was repeated for a Sp dCas9 construct, as well as a 1:1 equimolar mix of Sp Cas9 and Sp dCas9 constructs.

Note that the use of a mix of Sp Cas9 and Sp dCas9 DNA constructs measures the efficiency of encapsulating ≤1 DNA construct per emulsion droplet. In perfect efficiency of encapsulating only ≤1 DNA construct per droplet, none of the Sp dCas9 sequences detected via nanopore sequencing at the end of the assay for the mixed DNA input condition should be cleaved. In non-perfect efficiency, some Sp dCas9 DNA constructs might be cleaved since some would be exposed to active Sp Cas9 in the same droplet. As such, if the detection rate of cleaved Sp dCas9 constructs in the assay of mixed Sp Cas9 and Sp dCas9 constructs occurs at a very low rate comparable to the expected rate of random sequencing errors from long-read nanopore sequencing, the data will indicate that ≤1 sequence copy was encapsulated in each emulsion droplet under these conditions. This example demonstrates the full workflow of our invention as shown in Figure 1.

The generated emulsion IVTT mixtures were then incubated for 4 h at 37 °C for IVTT to proceed, followed by 65 °C for 15 min to inactivate the proteins.

The emulsion IVTT mixture was then treated as described above to break the emulsions. 20 mM EDTA (pH 8.0) inhibitor was added to the emulsion and mixed briefly via vortexing. The emulsion mixtures were then centrifuged at 13000 g for 5 min at room temperature. The upper oil layer was removed. 1 mL of water-saturated diethyl ether was added to the remaining aqueous layer, vortexed, and the upper solvent layer was removed; this step was repeated once. The remaining aqueous layer was centrifuged under vacuum at room temperature for 5 min, then treated with RNase cocktail and Proteinase K to remove excess RNA and proteins from the IVTT reaction at 37 °C for 30 min. The DNA from all the IVTT reactions were then purified individually with a commercial column purification kit (DNA Clean and Concentrator-5, Zymo Research) following the manufacturer's instructions.

The purified DNA from the IVTT reactions were then treated for nanopore sequencing using the ONT SQK-LSK109 ligation sequencing kit and barcoded individually using the ONT EXP-NBD104 PCR-Free native barcoding expansion kit. Single-molecule long-read nanopore sequencing was then performed on the DNA libraries using the ONT MinION Mk1B sequencing device. The nanopore sequencing results were then filtered for quality and analyzed using publicly available bioinformatics tools, followed by the analytic approach disclosed in this invention.

The Sp Cas9 emulsion IVTT nanopore sequencing reads show a mix of cleaved and uncleaved construct fragments detected (Figure 12), demonstrating that the Sp Cas9 is active upon a fraction of the target (as per demonstrated in bulk reactions). The Sp dCas9 emulsion IVTT nanopore sequencing reads show up overwhelmingly as uncleaved construct fragments, demonstrating that the Sp dCas9 is inactive majority of the time, as expected (Figure 13); the small minority of reads classified as "cleaved" Sp dCas9 construct fragments are likely the result of truncated/incomplete reads during nanopore sequencing and/or random DNA shearing events.

Note that some reads in the Sp Cas9-only and Sp dCas9-only sublibraries were mapped to the wrong sequences e.g. reads mapping to Sp dCas9 instead of Sp Cas9 within a Sp Cas9-only sublibrary, likely a result of sequencing error on the sequencing device or error in the de-multiplexing of barcoded nanopore sequencing reads, and were thus classified as mis-assigned and depicted as such in the plots.

The nanopore sequencing reads generated from the emulsion IVTT reaction with a 1:1 mix of Sp Cas9 and Sp dCas9 constructs added at limiting concentrations show an approximately equal distribution of Sp Cas9 and Sp dCas9 mapped reads as expected (Figure 14). The Sp Cas9 mapped reads show a nearly equal split of cleaved and uncleaved fragments, while a large majority of Sp dCas9 mapped reads are classified as uncleaved. A minority of Sp dCas9 mapped reads are classified as cleaved, as may have partly arisen from errors in sequencing or de-multiplexing, since these sequencing errors are known to occur on the sequencing devices when mixtures of fragments are sequenced, or via errors in cross-contamination of the enzymatic complexes, as can be further reduced by technical optimization within the inventive concept. Together, this example embodies and demonstrates the disclosed invention, where graded levels of enzymatic activities of variants can be directly counted and determined on the single-molecule basis.

## Claims

1. A method comprising the steps of:

   a) segregating a plurality of polynucleotide constructs into compartments, wherein each compartment comprises a single polynucleotide construct, wherein each polynucleotide construct comprises

   i) a first polynucleotide sequence encoding a nucleic acid modifying enzyme or a variant thereof, operably linked to a first promoter; and
   ii) a second polynucleotide sequence comprising a DNA target or a DNA template encoding an RNA target, wherein when the second polynucleotide sequence comprises a DNA template encoding an RNA target, said RNA target is co-expressed contiguously with the nucleic acid modifying enzyme as a single RNA transcript, driven by the first promoter;

   and wherein the plurality of the polynucleotide constructs encode different variants of the nucleic acid modifying enzyme, and/or different DNA or RNA targets;
   b) subjecting the compartments to conditions which allow *in vitro* expression of RNAs and proteins;
   c) subjecting the plurality of the compartments to conditions which allow the modification of DNA/RNA targets by nucleic acid modifying enzymes which have modification activity towards said DNA or RNA targets, thereby producing a population of DNA/RNA molecules that comprises one or more of the following:

   i. polynucleotide constructs and/or RNA transcripts or fragments thereof that have been modified by the nucleic acid modifying enzyme(s);
   ii. polynucleotide constructs and/or RNA transcripts which have not been modified by the nucleic acid modifying enzyme(s);

   d) harvesting the population of DNA/RNA molecules produced in step (c) and subjecting the same to single molecule sequencing;
   e) detecting and counting the DNA/RNA molecules referred to in step c)i and c)ii based on the sequencing results.

2. The method according to claim 1, wherein the nucleic acid modifying enzyme is an RNA-guided nucleic acid modifying enzyme, each compartment further comprises a guide RNA or a nucleotide template encoding the same; or wherein the nucleic acid modifying enzyme is an RNA-guided nucleic acid modifying enzyme, each polynucleotide further comprises a third polynucleotide sequence encoding a variant guide RNA (gRNA); and wherein the plurality of the polynucleotide constructs encode different variants of the nucleic acid modifying enzyme, and/or different DNA or RNA targets, and/or different gRNAs.

3. A method comprising the steps of:

a) segregating a plurality of polynucleotide constructs into compartments, wherein each compartment comprises a single polynucleotide construct, wherein each polynucleotide construct comprises:

i) a first polynucleotide sequence encoding a guide RNA (gRNA) operably linked to a first promoter;
ii) a second polynucleotide sequence comprising a DNA target or a DNA template encoding an RNA target, wherein when the second polynucleotide sequence comprises a DNA template encoding an RNA target, said RNA target is co-expressed contiguously with the gRNA as a single RNA transcript, driven by the first promoter;

wherein the plurality of the polynucleotide constructs encode different gRNAs, and/or different DNA or RNA targets; and wherein each compartment further comprises an RNA-guided nucleic acid modifying enzyme or a variant thereof or a nucleotide template encoding the same;
b) subjecting the compartments to conditions which allow in vitro transcription and/or translation of RNAs and proteins;
c) subjecting the compartments to conditions which allow the modification of DNA and/or RNA targets by RNA-guided nucleic acid modifying enzymes which have functional activity towards said DNA or RNA targets in the presence of a gRNA, thereby producing a population of DNA/RNA molecules that comprises one or more of the following:

i. polynucleotide constructs and/or RNA transcripts or fragments thereof that have been modified by the nucleic acid modifying enzyme(s);
ii. polynucleotide constructs and/or RNA transcripts which have not been modified by the nucleic acid modifying enzyme(s);

d) harvesting the population of DNA/RNA molecules produced in step (c) and subjecting the same to single molecule long-read sequencing;
e) detecting and counting the DNA/RNA molecules referred to in step c)i and/or c)ii based on the sequencing results.

4. The method of any of claims 1 to 3, wherein the method further comprises evaluating the modifying activity of one or more nucleic acid modifying enzymes against one or more of the DNA/RNA targets, by calculating the number of polynucleotide constructs and/or RNA transcripts that have been modified by the nucleic acid modifying enzyme ($\Sigma counts^{modified}$), and comparing it against the number of polynucleotide constructs and/or RNA transcripts that have not been modified by the nucleic acid modifying enzymes ($\Sigma counts^{unmodified}$), or against the total number of polynucleotide constructs and/or RNA transcripts $\Sigma country^{modified}/\Sigma counts^{unmodified}$ (); optionally wherein the enzymatic activity is represented by a value calculated using any one of the following formulas:

1)

$$enzymatic\ activity \approx \sum counts^{modified} / \sum counts^{unmodified} ;$$

2)

$$enzymatic\ activity \approx \sum counts^{modified} / \sum counts^{modified+unmodified} .$$

5. The method of any of claims 1 to 4, wherein step d) further comprises breaking the compartments by physical or chemical methods; and/or wherein step d) further comprises purifying the harvested DNA/RNA molecules to remove excess DNA, RNA and/or proteins from the reaction; or wherein the harvested population of DNA/RNA molecules are not subjected to further modifications before being subjected to the single molecule sequencing reaction, except for modifications required of the single molecule sequencing; or wherein the detection and counting of the DNA/RNA molecules which have or have not been modified by the nucleic acid modifying enzyme(s) is based only on data generated during the single molecule sequencing and does not require further modifications or processing of the DNA/RNA molecules.

6. The method of claim 5, wherein the modification activity is cleavage activity, and the detection and calculation of modified and unmodified polynucleotide constructs or RNA transcripts are achieved by aligning sequencing reads of the DNA/RNA molecules against a reference sequence which contains a window of cleavage sites for the nucleic acid modifying enzyme(s), wherein

i) when the sequencing read of a DNA/RNA molecule is mapped across both 5' and 3' of the window of cleavage sites, the DNA/RNA molecule is an unmodified polynucleotide construct or RNA target;
ii) when the end of the sequencing read of a DNA/RNA molecule is mapped to within the window of cleavage sites, the DNA/RNA molecule is a modified polynucleotide construct or RNA target;
iii) when the end of the sequencing read of a DNA/RNA molecule does not map within the window of cleavage sites, nor across both 5' and 3' of the window of cleavage sites, the DNA/RNA molecule is non-informative and is not used for the measurement of modification activity.

7. The method of claim 1 wherein the plurality of the polynucleotide constructs encode different variants of the nucleic acid modifying enzyme and different DNA or RNA targets.

**Patentansprüche**

1. Ein Verfahren umfassend die Schritte:

a) Aufteilen einer Vielzahl von Polynukleotidkonstrukten in Kompartimente, wobei jedes Kompartiment ein einzelnes Polynukleotidkonstrukt umfasst, wobei jedes Polynukleotidkonstrukt umfasst

i) eine erste Polynukleotidsequenz, die für ein nukleinsäuremodifizierendes Enzym oder eine Variante davon kodiert und funktionsfähig an einen ersten Promotor gebunden ist; und
ii) eine zweite Polynukleotidsequenz umfassend ein DNA-Ziel oder eine DNA-Vorlage, die für ein RNA-Ziel kodiert, wobei, wenn die zweite Polynukleotidsequenz eine DNA-Vorlage umfasst, die für ein RNA-Ziel kodiert, das RNA-Ziel zusammenhängend mit dem nukleinsäuremodifizierenden Enzym als ein einziges RNA-Transkript koexprimiert wird, gesteuert durch den ersten Promotor;

und wobei die Vielzahl der Polynukleotidkonstrukte für unterschiedliche Varianten des nukleinsäuremodifizierenden Enzyms, und/oder für unterschiedliche DNA- oder RNA-Ziele kodiert;
b) Aussetzen der Kompartimente Bedingungen, die die *in vitro* Expression von RNAs und Proteinen erlauben;
c) Aussetzen der Vielzahl von Kompartimenten Bedingungen, die die Modifikation von DNA-/RNA-Zielen durch nukleinsäuremodifizierende Enzyme, die eine modifizierende Aktivität gegenüber den DNA- oder RNA-Zielen aufweisen, ermöglichen, wodurch eine Population von DNA-/RNA-Molekülen erzeugt wird, die eines oder mehrere der Folgenden umfasst:

i. Polynukleotidkonstrukte und/oder RNA-Transkripte oder Fragmente davon, die durch das/die nukleinsäuremodifizierende(n) Enzym(e) modifiziert wurden;
ii. Polynukleotidkonstrukte und/oder RNA-Transkripte, die nicht durch das/die nukleinsäuremodifizierende(n) Enzym(e) modifiziert wurden;

d) Ernten der in Schritt (c) erzeugten Population von DNA-/RNA-Molekülen und Unterziehen derselben einer Einzelmolekülsequenzierung;
e) Erkennen und Zählen der in Schritt c)i und c)ii genannten DNA-/RNA-Moleküle auf Grundlage der Sequenzierungsergebnisse.

2. Das Verfahren gemäß Anspruch 1, wobei das nukleinsäuremodifizierende Enzym ein RNA-gesteuertes nukleinsäuremodifizierendes Enzym ist, wobei jedes Kompartiment ferner eine Guide-RNA oder eine Nukleotid-Vorlage, die für diese kodiert, umfasst; oder wobei das nukleinsäuremodifizierende Enzym ein RNA-gesteuertes nukleinsäuremodifizierendes Enzym ist, wobei jedes Polynukleotid ferner eine dritte Polynukleotidsequenz umfasst, die für eine Guide-RNA-Variante (gRNA) kodiert; und wobei die Vielzahl der Polynukleotidkonstrukte unterschiedliche Varianten des nukleinsäuremodifizierenden Enzyms, und/oder unterschiedliche DNA- oder RNA-Ziele, und/oder unterschiedliche gRNAs kodiert.

**3.** Ein Verfahren umfassend die Schritte:

a) Aufteilen einer Vielzahl von Polynukleotidkonstrukten in Kompartimente, wobei jedes Kompartiment ein einzelnes Polynukleotidkonstrukt umfasst, wobei jedes Polynukleotidkonstrukt umfasst:

i) eine erste Polynukleotidsequenz, die für eine Guide-RNA (gRNA) kodiert und funktionsfähig an einen ersten Promotor gebunden ist;
ii) eine zweite Polynukleotidsequenz umfassend ein DNA-Ziel oder eine DNA-Vorlage, die für ein RNA-Ziel kodiert, wobei, wenn die zweite Polynukleotidsequenz eine DNA-Vorlage umfasst, die für ein RNA-Ziel kodiert, das RNA-Ziel zusammenhängend mit der Guide-RNA als ein einziges RNA-Transkript koexprimiert wird, gesteuert durch den ersten Promotor;

wobei die Vielzahl der Polynukleotidkonstrukte für unterschiedliche gRNAs, und/oder für unterschiedliche DNA- oder RNA-Ziele kodieren; und wobei jedes Kompartiment ferner ein RNA-gesteuertes nukleinsäuremodifizierendes Enzym oder eine Variante davon oder eine Nukleotid-Vorlage, die diese kodiert, umfasst.
b) Aussetzen der Kompartimente Bedingungen, die die in vitro Transkription und/oder Translation von RNAs und Proteinen erlauben;
c) Aussetzen der Kompartimente Bedingungen, die die Modifikation von DNA- und/oder RNA-Zielen durch RNA-gesteuerte nukleinsäuremodifizierende Enzyme, die eine modifizierende Aktivität gegenüber den DNA- oder RNA-Zielen in Gegenwart einer gRNA aufweisen, ermöglichen, wodurch eine Population von DNA-/RNA-Molekülen erzeugt wird, die eines oder mehrere der Folgenden umfasst:

i. Polynukleotidkonstrukte und/oder RNA-Transkripte oder Fragmente davon, die durch das/die nukleinsäuremodifizierende(n) Enzym(e) modifiziert wurden;
ii. Polynukleotidkonstrukte und/oder RNA-Transkripte, die nicht durch das/die nukleinsäuremodifizierende(n) Enzym(e) modifiziert wurden;

d) Ernten der in Schritt (c) erzeugten Population von DNA-/RNA-Molekülen und Unterziehen derselben einer Einzelmolekül-Long-Read-Sequenzierung;
e) Erkennen und Zählen der in Schritt c)i und/oder c)ii genannten DNA-/RNA-Moleküle auf Grundlage der Sequenzierungsergebnisse.

**4.** Das Verfahren gemäß einem der Ansprüche 1 bis 3, wobei das Verfahren ferner das Bewerten der modifizierenden Aktivität eines oder mehrerer der nukleinsäuremodifizierenden Enzyme gegenüber einem oder mehreren der DNA-/RNA-Ziele, durch Berechnen der Anzahl an Polynukleotidkonstrukten und/oder RNA-Transkripten, die durch das nukleinsäuremodifizierende Enzym modifiziert wurden ($\Sigma coubts^{modified}$), und Vergleichen mit der Anzahl an Polynukleotidkonstrukten und/oder RNA-Transkripten, die nicht durch die nukleinsäuremodifizierenden Enzyme modifiziert wurden ($\Sigma counts^{unmodified}$), oder mit der Gesamtzahl der Polynukleotidkonstrukte und/oder RNA-Transkripte $\Sigma counts^{modified}/\Sigma counts^{unmodified}$ (), umfasst; wobei gegebenenfalls die Enzymaktivität durch einen unter Verwendung einer der folgenden Formeln berechneten Wertes repräsentiert wird:

1)

$$enzymatic\ activity \approx \sum counts^{modified} / \sum counts^{unmodified};$$

2)

$$enzymatic\ activity \approx \sum counts^{modified} / \sum counts^{modified+unmodified}.$$

**5.** Das Verfahren gemäß einem der Ansprüche 1 bis 4, wobei Schritt d) ferner das Aufbrechen der Kompartimente durch physikalische oder chemische Verfahren umfasst; und/oder wobei Schritt d) ferner das Reinigen der geernteten DNA-/RNA-Moleküle umfasst, um überschüssige DNA, RNA und/oder Proteine aus der Reaktion zu entfernen; oder wobei die geerntete Population von DNA-/RNA-Molekülen keinen weiteren Modifikationen unterworfen wird, bevor sie der Einzelmolekülsequenzierungsreaktion ausgesetzt wird, mit Ausnahme der für die Einzelmolekülsequenzierung erforderlichen Modifikationen; oder wobei das Erkennen und Zählen der DNA-/RNA-Moleküle, die durch das/die nukleinsäuremodifizierende(n) Enzym(e) modifiziert oder nicht modifiziert wurden, nur auf Daten basiert, die während der Einzelmolekülsequenzierung erzeugt wurden und keine weiteren Modifikationen oder Behandlungen der DNA-/RNA-Moleküle erfordern.

6. Das Verfahren gemäß Anspruch 5, wobei die Modifikationsaktivität eine Spaltungsaktivität ist, und das Erkennen und die Berechnung modifizierter und unmodifizierter Polynukleotidkonstrukte oder RNA-Transkripte durch Abgleichen der Sequenzierungsabschnitte der DNA-/RNA-Moleküle mit einer Referenzsequenz erfolgen, die ein Fenster mit Spaltstellen für das/die nukleinsäuremodifizierende(n) Enzym(e) enthält, wobei

i) wenn der Sequenzierungsabschnitt eines DNA-/RNA-Moleküls 5' und 3' über das Fenster mit den Spaltstellen hinaus zugeordnet wird, das DNA-/RNA-Molekül ein unmodifiziertes Polynukleotidkonstrukt oder RNA-Ziel ist;
ii) wenn das Ende des Sequenzierungsabschnitts eines DNA-/RNA-Moleküls innerhalb des Fensters mit den Spaltstellen zugeordnet wird, das DNA/RNA-Molekül ein modifiziertes Polynukleotidkonstrukt oder RNA-Ziel ist;
iii) wenn das Ende des Sequenzierungsabschnitts eines DNA-/RNA-Moleküls weder innerhalb des Fensters der Spaltstellen liegt, noch 5' und 3' über das Fenster der Spaltstellen hinaus zugeordnet werden kann, das DNA-/RNA-Molekül nicht aussagekräftig und nicht zur Messung der Modifikationsaktivität verwendet wird.

7. Das Verfahren gemäß Anspruch 1, wobei die Vielzahl der Polynukleotidkonstrukte unterschiedliche Varianten des nukleinsäuremodifizierenden Enzyms und unterschiedliche DNA- oder RNA-Ziele kodiert.

**Revendications**

1. Procédé comprenant les étapes consistant à :

a) ségréger une pluralité de constructions polynucléotidiques dans des compartiments, sachant que chaque compartiment comprend une seule construction polynucléotidique, sachant que chaque construction polynucléotidique comprend

i) une première séquence polynucléotidique codant pour une enzyme de modification des acides nucléiques ou une variante de celle-ci, liée de manière opérationnelle à un premier promoteur ; et
ii) une deuxième séquence polynucléotidique comprenant une cible d'ADN ou une matrice d'ADN codant pour une cible d'ARN, sachant que lorsque la deuxième séquence polynucléotidique comprend une matrice d'ADN codant pour une cible d'ARN, ladite cible d'ARN est coexprimée de manière contiguë avec l'enzyme de modification des acides nucléiques sous la forme d'un unique transcrit d'ARN, sous le contrôle du premier promoteur ;

et sachant que la pluralité des constructions polynucléotidiques codent pour différentes variantes de l'enzyme de modification des acides nucléiques, et/ou différentes cibles d'ADN ou d'ARN ;
b) soumettre les compartiments à des conditions permettant l'expression *in vitro* d'ARN et de protéines ;
c) soumettre la pluralité des compartiments à des conditions permettant la modification de cibles d'ADN/ARN par des enzymes de modification des acides nucléiques ayant une activité de modification à l'égard desdites cibles d'ADN ou d'ARN, produisant ainsi une population de molécules d'ADN/ARN comprenant un ou plusieurs des termes suivants :

i. des constructions polynucléotidiques et/ou des transcrits d'ARN ou fragments de ceux-ci ayant été modifiés par la ou les enzymes de modification des acides nucléiques ;
ii. des constructions polynucléotidiques et/ou des transcrits d'ARN n'ayant pas été modifiés par la ou les enzymes de modification des acides nucléiques ;

d) récolter la population de molécules d'ADN/ARN produite à l'étape (c) et la soumettre à un séquençage moléculaire simple ;
e) détecter et compter les molécules d'ADN/ARN mentionnées à l'étape c)i et/ou c)ii sur la base des résultats de séquençage.

**2.** Le procédé selon la revendication 1, sachant que l'enzyme de modification des acides nucléiques est une enzyme de modification des acides nucléiques guidée par ARN, chaque compartiment comprend en outre un ARN guide ou une matrice nucléotidique codant pour celui-ci ; ou sachant que l'enzyme de modification des acides nucléiques est une enzyme de modification des acides nucléiques guidée par ARN, chaque polynucléotide comprend en outre une troisième séquence polynucléotidique codant pour une variante d'ARN guide (ARNg) ; et sachant que la pluralité des constructions polynucléotidiques codent pour différentes variantes de l'enzyme de modification des acides nucléiques, et/ou différentes cibles d'ADN ou d'ARN, et/ou différents ARNg.

**3.** Procédé comprenant les étapes consistant à :

a) ségréger une pluralité de constructions polynucléotidiques dans des compartiments, sachant que chaque compartiment comprend une seule construction polynucléotidique, sachant que chaque construction polynucléotidique comprend

i) une première séquence polynucléotidique codant pour un ARN guide (ARNg) lié de manière opérationnelle à un premier promoteur ;
ii) une deuxième séquence polynucléotidique comprenant une cible d'ADN ou une matrice d'ADN codant pour une cible d'ARN, sachant que lorsque la deuxième séquence polynucléotidique comprend une matrice d'ADN codant pour une cible d'ARN, ladite cible d'ARN est coexprimée de manière contiguë avec l'ARNg sous la forme d'un unique transcrit d'ARN, sous le contrôle du premier promoteur ;

sachant que la pluralité des constructions polynucléotidiques codent pour différents ARNg, et/ou différentes cibles d'ADN ou d'ARN ; et sachant que chaque compartiment comprend en outre une enzyme de modification des acides nucléiques guidée par ARN ou une variante de celle-ci ou une matrice nucléotidique codant pour celle-ci ;
b) soumettre les compartiments à des conditions permettant la transcription et/ou traduction *in vitro* d'ARN et de protéines ;
c) soumettre les compartiments à des conditions permettant la modification de cibles d'ADN et/ou d'ARN par des enzymes de modification des acides nucléiques guidées par ARN ayant une activité fonctionnelle à l'égard desdites cibles d'ADN ou d'ARN en la présence d'un ARNg, produisant ainsi une population de molécules d'ADN/ARN comprenant un ou plusieurs des termes suivants :

i. des constructions polynucléotidiques et/ou des transcrits d'ARN ou fragments de ceux-ci ayant été modifiés par la ou les enzymes de modification des acides nucléiques ;
ii. des constructions polynucléotidiques et/ou des transcrits d'ARN n'ayant pas été modifiés par la ou les enzymes de modification des acides nucléiques ;

d) récolter la population de molécules d'ADN/ARN produite à l'étape (c) et la soumettre à un séquençage moléculaire simple à lecture longue ;
e) détecter et compter les molécules d'ADN/ARN mentionnées à l'étape c)i et/ou c)ii sur la base des résultats de séquençage.

**4.** Le procédé de l'une quelconque des revendications 1 à 3, sachant que le procédé comprend en outre l'évaluation de l'activité de modification d'une ou de plusieurs enzymes de modification des acides nucléiques vis-à-vis d'une ou de plusieurs cibles d'ADN/ARN, en calculant le nombre de constructions polynucléotidiques et/ou de transcrits d'ARN ayant été modifiés par l'enzyme de modification des acides nucléiques ($\Sigma counts^{modified}$) ($\Sigma comptes^{modifiés}$), et en le comparant avec le nombre de constructions polynucléotidiques et/ou de transcrits d'ARN n'ayant pas été modifiés par les enzymes de modification des acides nucléiques ($\Sigma counts^{unmodified}$) ($\Sigma comptes^{nonmodifiés}$), ou avec le nombre total de constructions polynucléotidiques et/ou de transcrits d'ARN ($\Sigma counts^{modified}/\Sigma counts^{unmodified}$) ($\Sigma comptes^{modifiés}|\Sigma comptes^{non\ modifiés}$) ; facultativement sachant que l'activité enzymatique est représentée par une valeur calculée moyennant l'une quelconque des formules suivantes :

1)

$$enzymatic\ activity \approx \sum counts^{modified} / \sum counts^{unmodified} \;;$$

2)

$$enzymatic\ activity \approx \sum counts^{modified} / \sum counts^{modified+unmodified} \;.$$

(1)

$$Activité\ enzymatique \approx \sum comptes^{modifiés} / \sum comptes^{non\ modifiés}$$

(2)

$$Activité\ enzymatique \approx \sum comptes^{modifiés} / \sum comptes^{modifiés+non\ modifiés}$$

**5.** Le procédé de l'une quelconque des revendications 1 à 4, sachant que l'étape d) comprend en outre la rupture des compartiments par des procédés physiques ou chimiques ; et/ou sachant que l'étape d) comprend en outre la purification des molécules d'ADN/ARN récoltées pour l'ADN, l'ARN et/ou les protéines excédentaires issus de la réaction ; ou sachant que la population récoltée de molécules d'ADN/ARN n'est pas soumise à des modifications supplémentaires avant d'être soumise à la réaction de séquençage moléculaire simple, sauf les modifications requises par le procédé de séquençage moléculaire simple ; ou sachant que la détection et le comptage des molécules d'ADN/ARN ayant ou n'ayant pas été modifiées par la ou les enzymes de modification des acides nucléiques sont basés uniquement sur des données générées lors du séquençage moléculaire simple et ne nécessitent pas de modifications ou traitement supplémentaires des molécules d'ADN/ARN.

**6.** Le procédé de la revendication 5, sachant que l'activité de modification est une activité de clivage, et la détection et le calcul de constructions polynucléotidiques ou de transcrits d'ARN modifiés et non modifiés sont réalisés en alignant des lectures de séquençage des molécules d'ADN/ARN contre une séquence de référence contenant une fenêtre de sites de clivage pour la ou les enzymes de modification des acides nucléiques, sachant que

i) lorsque la lecture de séquençage d'une molécule d'ADN/ARN s'aligne à la fois sur 5' et 3' de la fenêtre de sites de clivage, la molécule d'ADN/ARN est une construction polynucléotidique ou une cible d'ARN non modifiée ;
ii) lorsque l'extrémité de la lecture de séquençage d'une molécule d'ADN/ARN s'aligne à l'intérieur de la fenêtre de sites de clivage, la molécule d'ADN/ARN est une construction polynucléotidique ou une cible d'ARN modifiée ;
iii) lorsque l'extrémité de la lecture de séquençage d'une molécule d'ADN/ARN ne s'aligne ni à l'intérieur de la fenêtre de sites de clivage, ni à la fois sur 5' et 3' de la fenêtre de sites de clivage, la molécule d'ADN/ARN est non informative et n'est pas utilisée pour la mesure de l'activité de modification.

**7.** Le procédé de la revendication 1, sachant que la pluralité de constructions polynucléotidiques codent pour différentes variantes de l'enzyme de modification des acides nucléiques et différentes cibles d'ADN ou d'ARN.

DNA variant library

Emulsification in droplets

*In vitro* transcription and translation

Single-molecule long-read sequencing

Enzymatic activities

Endonuclease breakage

Inactive, unmodified

Sequence(s) change; chemical mod

Direct electrochemical signals corresponding to DNA/RNA sequences and any epigenetic modifications

Variant identities

Target identities

Variant 1;Target identities= A A A A A A GA GA A A A ...
Variant 2;Target identities= A A A A A A A A A A A A A ...
Variant 3;Target identities= A nil A nil A nil nil nil A A nil ...
Variant 4;Target identities= A A A A A A mA A A A mA A A ...

.
.
.

For each variant,
Endonuclease enzymatic activity ≈ ($\Sigma$counts$^{cleaved}$ / $\Sigma$counts$^{uncleaved}$)
Modifier enzymatic activity ≈ ($\Sigma$counts$^{modified}$ / $\Sigma$counts$^{unmodified}$)

**Figure 1**

Single IVTT DNA constructs for DNA-targeting Cas:

| First Promoter | Cas Nuclease | First Terminator | Second Promoter | gRNA | Second Terminator | Target |

| First Promoter | gRNA | First Terminator | Second Promoter | Cas nuclease | Second Terminator | Target |

| First Promoter | gRNA | First Terminator | Target | Second Promoter | Cas nuclease | Second Terminator |

| First Promoter | Cas nuclease | First Terminator | Target | Second Promoter | gRNA | Second Terminator |

| Target | First Promoter | Cas Nuclease | First Terminator | Second Promoter | gRNA | Second Terminator |

| Target | First Promoter | gRNA | First Terminator | Second Promoter | Cas nuclease | Second Terminator |

Single IVTT DNA constructs for RNA-targeting Cas:

| First Promoter | Cas nuclease | Target | First Terminator | Second Promoter | gRNA | Second Terminator |

| First Promoter | Target | Cas nuclease | First Terminator | Second Promoter | gRNA | Second Terminator |

| First Promoter | Cas nuclease with target sequence encoded within | First Terminator | Second Promoter | gRNA | Second Terminator |

# Figure 2

**Figure 3**

**Figure 4**

Emulsion IVTT reaction with high Sp Cas9 DNA input: reads mapped

**Figure 5**

Emulsion IVTT reaction with high Sp dCas9 DNA input: reads mapped

**Figure 6**

Bulk IVTT reactions:

**1.** For Cas variants SpCas9, SaCas9, AsCpf1, LbCpf1, setup individual 100µl PURExpress reactions + 1µg template DNA.

**2.** Start 37°C incubation for the tubes and remove the corresponding tubes per timepoint to quench with 20 mM EDTA + RNase cocktail + Proteinase K, quench at 37°C for 30 min.

Split each 100µl reaction master mix into 5 x 20µl reactions for each time point:
t=0h    t=1h    t=2h    t=3h    t=4h

**3.** SPRI bead cleanup of quenched IVTT reactions to obtain DNA fragments per timepoint per Cas species.

**4.** Pool DNA fragments by timepoints, then start ONT library preparation with ONT PCR-free ligation sequencing kit and ligate barcode adapters per timepoint batch for multiplexing.

**5.** Pool barcoded DNA fragments for a multiplexed library to be sequenced on the MinION in a single run.

t=0h    t=1h    t=2h
t=3h    t=4h

- ∿∿ DNA fragments
- ▒ barcode sequences
- ⬡ ONT adapters

**Figure 7**

**Figure 8**

Figure 9

Figure 10

Figure 11

40

Emulsion IVTT reaction with limiting Sp Cas9 DNA input: reads mapped

**Figure 12**

Emulsion IVTT reaction with limiting Sp dCas9 DNA input: reads mapped

**Figure 13**

Emulsion IVTT reaction with limiting Sp Cas9 and dCas9 (1:1) DNA input: reads mapped

Reads mapped
Sp dCas9 uncleaved
Sp dCas9 cleaved
Sp Cas9 uncleaved
Sp Cas9 cleaved

**Figure 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018118968 A1

**Non-patent literature cited in the description**

- **PALMER D. J.** ; **TURNER D. L** ; **NG P.** Production of CRISPR/Cas9-Mediated Self-Cleaving Helper-Dependent Adenoviruses.. *Mol Ther Methods Clin Dev*, 2019, vol. 13, 432-439
- **LI, H**. Minimap2: pairwise alignment for nucleotide sequences.. *Bioinformatics*, 2018, vol. 34, 3094-3100
- **DE COSTER, W. et al.** NanoPack: visualizing and processing long-read sequencing data.. *Bioinformatics*, 2018, vol. 34, 2666-2699
- **LI, H. et al.** The Sequence Alignment/Map format and SAMtools.. *Bioinformatics*, 2009, vol. 25, 2078-9
- **KOBOLDT, D.C. et al.** VarScan 2: Somatic mutation and copy number alteration discovery in cancer by exome sequencing.. *Genome Research*, 2012, vol. 22, 568-576
- **LIU, Q. et al.** Detection of DNA base modifications by deep recurrent neural network on Oxford Nanopore sequencing data.. *Nat Commun*, 2019, vol. 10 (1), 2449
- **LIU, Q. et al.** NanoMod: a computational tool to detect DNA modifications using Nanopore long-read sequencing data.. *BMC Genomics*, 2019, vol. 20 (1), 78
- **RAND, A. C. et al.** Mapping DNA methylation with high-throughput nanopore sequencing.. *Nat Methods*, 2017, vol. 14 (4), 411-413
- **SIMPSON, J. T. et al.** Detecting DNA cytosine methylation using nanopore sequencing.. *Nat Methods*, 2017, vol. 14 (4), 407-410